# EUROPÄISCHE PATENTANMELDUNG

(11) **EP 4 234 491 A1**
(43) Veröffentlichungstag der Anmeldung: **30.08.2023**
(21) Anmeldenummer: 22158664.7
(22) Anmeldetag: 24.02.2022
(51) Int. Cl.: C01B 32/80, C01B 32/40, C08J 11/10, C08J 11/12, C07C 263/10, C08G 18/00, C10B 53/07

(54) **VERFAHREN ZUR GASIFIKATION POLYMERER WERTSTOFFMATERIALIEN FÜR DIE EMISSIONSARME BEREITSTELLUNG VON FÜR DIE HERSTELLUNG VON PHOSGEN NUTZBAREM KOHLENMONOXID**

(71) Anmelder: Covestro Deutschland AG, 51373 Leverkusen (DE)
(72) Erfinder: Bulan, Andreas, 40764 Langenfeld (DE); Weber, Rainer, 51519 Odenthal (DE); Eiden, Stefanie, 50733 Köln (DE); Zabalza, Gaston, 10435 Berlin (DE)
(74) Vertreter: Levpat

(57) **Zusammenfassung**

Die Erfindung betrifft ein Verfahren gemäß Anspruch 1 zur emissionsarmen Herstellung von Kohlenmonoxid (CO) für die Herstellung von Phosgen, aus dem beispielsweise Polycarbonate oder organische Isocyanate und daraus Polyurethane hergestellt werden können, unter Einsatz eines Prozesses zur partiellen Oxidation unter Gasifikation polymerer Wertstoffmaterialien. Ferner betrifft die Erfindung eine dafür nutzbare Vorrichtung und die Verwendung des besagten Gasifikationsprozesses bzw. der polymeren Wertstoffmaterialien zur Bereitstellung von Kohlenmonoxid für die Herstellung von Phosgen.

## Beschreibung

Die Erfindung betrifft ein Verfahren zur emissionsarmen Herstellung von Kohlenmonoxid (CO) für die Herstellung von Phosgen, aus dem beispielsweise Polycarbonate oder organische Isocyanate und daraus Polyurethane hergestellt werden können, unter Einsatz eines Prozesses zur partiellen Oxidation unter Gasifikation polymerer Wertstoffmaterialien. Ferner betrifft die Erfindung eine dafür nutzbare Vorrichtung und die Verwendung des besagten Gasifikationsprozesses bzw. der polymeren Wertstoffmaterialien zur Bereitstellung von Kohlenmonoxid für die Herstellung von Phosgen.

Am Ende der Lebensdauer Polymermaterial-haltiger Produkte werden diese gegen neue Produkte ausgetauscht und in der Regel verschrottet. Die Gesamtmenge der resultierenden Kunststoffabfälle nimmt jedes Jahr zu. Etwa 60% der Gesamtmenge wird durch Verbrennen und auf der Deponie entsorgt. Bei der Verbrennung wird CO₂ in die Luft emittiert, das zu der globalen Erwärmung beiträgt. Der Plastikabfall auf den Deponien nimmt wegen seiner geringen Dichte ein großes Volumen ein und kann von dort auch zur allgemeinen Verschmutzung in Flüssen und Meeren beitragen. Aus diesem Grunde ist es wichtig, eine effiziente Recycling Methode zu entwickeln, mit der das Abfallproblem gelöst werden kann und auch gleichzeitig fossile Ressourcen eingespart werden können. Aufgrund des wachsenden Kunststoffmarktes ist es wichtig, für alle Polymerklassen Recycling Technologien zu entwickeln, um sowohl die CO₂ Emission zu vermindern und fossile Energien einzusparen.

Die Verfahren für das Recycling von Kunststoffabfällen lassen sich grob in 3 Kategorien unterteilen:
(1) mechanisches Recycling: Hierbei werden die Kunststoffabfälle so wie sie sind wieder eingeschmolzen und können dann wiederverwendet werden. Dies ist für Polyurethan Elastomere nicht möglich.
(2) chemisches und thermochemisches Recycling: Hierbei werden die Kunststoffabfälle in Monomere depolymerisiert oder zu kleineren Molekülen zersetzt, um nützliche chemische Rohstoffe zu gewinnen und
(3) thermisches Recycling, wobei die Kunststoffabfälle hierin zu Abgas und Wärmeenergie umgesetzt werden.

Die aus dem thermochemischen Recycling gewonnenen chemischen Rohstoffe können zur Synthese neuer Kunstharze oder anderer chemischer Produkte verwertet werden.

Ein Rohstoff für die Herstellung von Polyurethan-Polymeren oder Polycarbonat-Polymeren ist Kohlenmonoxid, welches mit Chlorgas zu Phosgen umgesetzt wird. So wird konventionell Kohlenmonoxid und Wasserstoff aus Erdgas bzw. aus Kohle mittels Reformingprozessen, und Chlor aus der Elektrolyse mit Strom, der unter Einsatz von fossilen Brennstoffen wie Öl, Kohle oder Erdgas hergestellt wird, gewonnen. Dabei entsteht eine erhebliche Menge an CO₂.

Es ist eine Aufgabe dieser Erfindung, ein nachhaltigeres Verfahren zur Kohlenmonoxid-Herstellung bereitzustellen. Unter "Nachhaltigkeit" eines Verfahrens versteht der Fachmann in Anwendung der durch die UN geprägte Nachhaltigkeits-Definition ("sustainable development") gemäß Brundtlandbericht der "Weltkommission für Umwelt und Entwicklung", dass durch die Ausführung des Verfahrens in der Gegenwart ein möglichst geringer bis gar kein Beitrag geleistet wird, dass künftige Generationen der Menschheit ihre eigenen Bedürfnisse nicht mehr befriedigen können, insbesondere Bedürfnisse mit Blick auf die Nutzung von Ressourcen wie z.B. fossiler Rohstoffe und insbesondere mit Blick auf die Schonung des Lebensraumes, wie z.B. den Schutz der Erdatmosphäre.

Die Erfindung hat somit zur Aufgabe, die Produktion von Kohlenmonoxid nachhaltiger als die aus dem Stand der Technik bekannten Produktionsmethoden zu gestalten, wobei diese Lösung möglichst einfach in bestehende Produktionslinien integrierbar sein soll.

In diesem Zusammenhang ist es eine Aufgabe der vorliegenden Erfindung, die CO₂-Emissionen bei der Bereitstellung von Kohlenmonoxid zu verringern.

Kohlenmonoxid kann aus unterschiedlichen nachhaltigen Prozessen erzeugt werden. So zum Beispiel durch einen Reformerprozess, in dem Erdgas aus einer fossilen Quelle zugeführt wird, wobei zusätzlich Bio-Methan, Kohlendioxid und/oder Wasserstoff zugeführt werden. Die notwendige Energie für den ablaufenden Reformerprozess wird aus der Verbrennung von Erdgas, Bio-Methan, Wasserstoff oder auch durch elektrische Beheizung erzeugt. Weiterhin kann CO aus der sogenannten Reverse-Water-Gas-Shift Reaktion aus CO₂ und H₂ erzeugt werden, wobei der Wasserstoff aus einer Wasserelektrolyse stammt. Ein weiter Option ist die elektrochemische CO₂ Reduktion zu CO.

Gemäß dem erfindungsgemäßen Verfahren erfolgt die Herstellung von CO jedoch aus gebrauchten Polymer-Abfallfraktionen und Sauerstoffgas durch Vergasung unter partieller Oxidation (im Folgenden auch als Gasifikation bezeichnet). Durch den Einsatz von Polymer-haltigen Abfallfraktionen, die beispielsweise überwiegend aus Polyurethan- oder Polycarbonat-haltigen Abfallfraktionen bestehen können (nachfolgend Polymer-Abfallfraktion genannt), können Polycarbonat Material oder Isocyanate und das daraus neue Polyurethan Material mit verbesserter Nachhaltigkeit hergestellt werden. Ein Teil des Kohlenstoffs wird dabei recycelt.

Durch das erfindungsgemäße Verfahren werden die Polymer-Abfallfraktion und Sauerstoff zur partiellen Oxidationsreaktion gebracht und zu einem Produktgasgemisch enthaltend Wasserstoff und CO und gegebenenfalls Nebenprodukte umgesetzt. Als Nebenprodukte gelten insbesondere Kohlenwasserstoffe mit 1 bis 8 Kohlenstoffatomen. Im Produktgasgemisch sind u.a. ebenso CO₂ und Wasserdampf vorhanden.

Als weiteres Nebenprodukt wird aus dem Gasifikation-Prozess eine nicht weiter umsetzbare Restfraktion erhalten.

Die für die Gasifikation benötigte Temperatur wird zumindest teilweise durch partielle Verbrennung (partielle Oxidation) der Polymer-Abfallfraktion mit einem sauerstoffhaltigen Gas erreicht.

Zur Lösung mindestens einer der vorgenannten Aufgaben eignet sich als erster Gegenstand der Erfindung ein Verfahren zur Bereitstellung von Kohlenmonoxid für die Herstellung von Phosgen, bevorzugt für die Herstellung von Phosgen zur Synthese organischer Isocyanatverbindung, enthaltend mindestens folgende Schritte
a) Bereitstellung eines sauerstoffhaltigen Gasstroms, enthaltend mindestens 50 Gew.-% Sauerstoffgas,
b) partielle Oxidation von Material, enthaltend mindestens eine polymere, organische Verbindung, wobei besagtes Material in einen Reaktor einer Vorrichtung eingebracht und dort zumindest durch Zufuhr des besagten sauerstoffhaltigen Gasstromes und von Wärme bei mindestens 400°C unter Bildung eines Produktgases behandelt und das resultierende Produktgas, gegebenenfalls nach mindestens einem weiteren partiellen Oxidationsschritt des Produktgases, als ein Kohlenmonoxid-haltiger Produktgas-Strom gemeinsam mit darin dispergiertem, partikelförmigem Feststoff aus der Vorrichtung ausgebracht wird;
c) der Kohlenmonoxid-haltige Produktgas-Strom einer Reinigung zugeführt wird, in der zumindest
   c1) der Kohlenmonoxid-haltige Produktgas-Strom zur Abtrennung von Feststoff einem Waschschritt zugeführt wird, worin
      (i) Wasser mit dem Kohlenmonoxid-haltigen Produktgas-Strom in Kontakt gebracht wird, wodurch der im Kohlenmonoxid-haltigen Produktgas-Strom dispergierte, partikelförmige Feststoff eine Schlacke bildet und diese Schlacke entfernt und abgeführt wird,
      (ii) der von partikelförmigem Feststoff gereinigte, Kohlenmonoxid-haltige Produktgas-Strom ausgebracht wird;
   c2) ein mittels Waschschritt von partikelförmigem Feststoff gereinigter Kohlenmonoxid-haltiger Produktgas-Strom in einem Trockenschritt zumindest einer Abtrennung von Wasser zugeführt, Wasser abgetrennt und der resultierende Kohlenmonoxid-haltige Produktgas-Strom ausgebracht wird,
   c3) ein mittels Abtrennung von Wasser gereinigter Kohlenmonoxid-haltiger Produktgas-Strom zumindest einer Abtrennung von Kohlendioxid zugeführt, Kohlendioxid abgetrennt und der resultierende Kohlenmonoxid-haltige Produktgas-Strom und Kohlendioxid ausgebracht wird;
   c4) ein mittels Abtrennung von Kohlendioxid gereinigter Kohlenmonoxid-haltiger Produktgas-Strom zumindest einer Trenneinheit zur Abtrennung von Kohlenmonoxid zugeführt, eine Abtrennung von Kohlenmonoxid durchgeführt und das resultierende Kohlenmonoxid und ein Wasserstoffgas-haltiges Restgas ausgebracht wird;
   c5) optional ein mittels besagter Trenneinheit abgetrenntes, Wasserstoffgas-haltiges Restgas einer Restgasbehandlung zugeführt, Wasserstoffgas abgetrennt und Wasserstoffgas und ein Endgas ausgebracht wird;
d) gegebenenfalls Phosgensynthese durch Umsetzung von zumindest Chlorgas und Kohlenmonoxid, wobei als Kohlenmonoxid mindestens das Kohlenmonoxid aus der Trenneinheit zur Abtrennung von Kohlenmonoxid umgesetzt und Phosgen ausgebracht wird.

Ein Feststoff ist bekanntermaßen "partikelförmig", wenn er in Form eines körnigen Gemenges aus einer Vielzahl an losen, festförmigen Partikeln des besagten Stoffes vorliegt, das wiederum sogenannte Körner umfasst. Ein Korn ist eine Bezeichnung für die partikulären Bestandteile von Pulvern (Körner sind die losen, festförmigen Partikel), Stäuben (Körner sind die losen festförmigen Partikel), Granulaten (lose, festförmige Partikel sind Agglomerate aus mehreren Körnern) und anderen körnigen Gemengen.

Eine "organische Verbindung" enthält mindestens eine kovalente Kohlenstoff-Wasserstoff-Bindung im Molekül. Ein organisches Isocyanat ist demnach ein organischer Stoff, der als chemische Verbindung mindestens eine Isocyanatgruppe und mindestens eine kovalente Kohlenstoff-Wasserstoff-Bindung im Molekül enthält. Ein organisches Amin ist *mutatis mutandis* definiert.

Eine "polymere Verbindung" ist ein Molekül mit einer relativen Molmasse (Mw) von mindestens 2000 g/mol, dessen chemische Struktur überwiegend sich mehrfach wiederholende Struktureinheiten umfasst, die sich von einem oder mehreren verschiedenen Molekülen geringerer relativer Molmasse ableiten. Bei den im Rahmen dieser Anmeldung für Polymere bzw. polymere Verbindungen angegebenen mittleren Molmassen handelt es sich - sofern nicht explizit anders gekennzeichnet - stets um gewichtsmittlere Molmassen Mw, die grundsätzlich mittels Gelpermeationschromatographie mit Hilfe eines RI-Detektors bestimmbar sind, wobei die Messung zweckmäßig gegen einen externen Standard erfolgt.

Unter "regenerativer Energie" versteht der Fachmann Energie aus einer Energiequelle, die sich nicht erschöpft, wie z.B. Windenergie, Wasserenergie, Bioenergie (z.B. Verstromung von Biogas oder Biomasse) oder Sonnenenergie. Als regenerative Energie eignen sich daher ganz besonders bevorzugt wahlweise Windkraft, Sonnenenergie, Wasserkraft oder Mischungen daraus.

Ein "Reaktor" ist ein Volumen, in dem eine chemischen Umwandlung, z.B. eine partielle Oxidation von polymerer, organischer Verbindung eines Materials, stattfindet. Dies kann für die partielle Oxidation beispielsweise das Volumen eines beheizten Gefäßes sein, in dem sich das Material befindet.

Der bereitgestellte sauerstoffhaltige Gasstrom, enthält mindestens 50 Gew.-% Sauerstoffgas. Vorzugsweise enthält der sauerstoffhaltige Gasstrom mindestens 60 Gew.-% Sauerstoffgas, weiter bevorzugt mindestens 75 Gew.-% Sauerstoffgas.

Das dafür benötigte Sauerstoffgas kann z.B. einer Wasserelektrolyse oder einer Luftzerlegungsanlage, die den Stickstoff insbesondere bei der Herstellung von Ammoniak liefert, entnommen werden. Somit kann der Wertschöpfungskreislauf der genannten Polymere bzgl. der Kohlenstoffs aus dem Kohlenmonoxid geschlossen werden. Im Rahmen einer bevorzugten Ausführungsform des Verfahrens, wird zur Bereitstellung des sauerstoffhaltigen Gasstroms eine Elektrolyse von Wasser unter Erhalt von Sauerstoffgas und Wasserstoffgas durchgeführt und das Sauerstoffgas aus dieser Elektrolyse (22) zur Bereitstellung des sauerstoffhaltigen Gasstromes genutzt.

Die Wasserelektrolyse kann mit Anlagen nach dem Stand der Technik durchgeführt werden. Bekannt und kommerziell erhältlich sind technische Anlagen zur alkalischen Wasserelektrolyse als auch zur Polymerelektrolyt-basierten Elektrolyse, der so genannten PEM-Elektrolyse. Die Prinzipien der Wasserelektrolyse sind beispielhaft in Kapitel 6.3.4 in Volkmar M. Schmidt in "Elektrochemische Verfahrenstechnik" (2003 Wiley-VCH-Verlag; ISBN 3-527-29958-0) beschrieben.

Neben der Einbringung des sauerstoffhaltigen Gasstroms kann es erfindungsgemäß bevorzugt sein, zusätzlich gasförmiges Wasser in den mindestens einen Reaktor der partiellen Oxidation einzubringen und die partielle Oxidation des besagten Materials in Gegenwart einer Mischung von Sauerstoffgas und gasförmigem Wasser durchzuführen. Dabei hat es sich als besonders bevorzugt erwiesen, wenn dem Reaktor zusätzlich gasförmiges Wasser zugeführt wird, in einem Gewichtsverhältnis von gasförmigem Wasser zu Sauerstoffgas des sauerstoffhaltigen Gasstromes von mindestens 0,2.

Das gasförmige Wasser und der sauerstoffhaltige Gasstrom können separat voneinander in den Reaktor eingebracht werden oder das gasförmige Wasser kann vor der Einbringung in den Reaktor zu dem sauerstoffhaltigen Gaststrom gemischt und mit diesem gemeinsam als dessen Komponente in den Reaktor eingebracht werden.

Das in den Reaktor eingebrachte Material enthält erfindungsgemäß mindestens eine polymere, organische Verbindung. Die partielle Oxidation sollte in dem Reaktor möglichst gleichmäßig und selektiv stattfinden. Eine Steigerung dieser Parameter kann erzielt werden, wenn im Rahmen einer bevorzugten Ausführungsform jeweils bezogen auf den Zeitpunkt vor der Einbringung das Gewichtsverhältnis des im sauerstoffhaltigen Gasstrom enthaltenen Sauerstoffgases zur polymeren, organischen Verbindung innerhalb eines Gewichtsverhältnisbereiches von 0,4 zu 1,0 bis 1,2 zu 1,0, bevorzugt von 0,6 zu 1,0 bis 0,9 zu 1,0 in den Reaktor eingebracht werden.

Es hat sich als vorteilhaft erwiesen, wenn das besagte Material partikelförmig in Form festförmiger Teilchen (insbesondere in Form eines körnigen Gemenges) in den Reaktor eingebracht wird. Ein körniges Gemenge desbesagten Materials wird aus einer Vielzahl an losen, festförmigen Partikeln des besagten Materials gebildet, die wiederum sogenannte Körner umfassen. Ein Korn ist eine Bezeichnung für die partikulären Bestandteile von Pulvern (Körner sind die losen, festförmigen Partikel), Stäuben (Körner sind die losen festförmigen Partikel), Granulaten (lose, festförmige Partikel sind Agglomerate aus mehreren Körnern) und anderen körnigen Gemengen.

Vorzugsweise weisen die festförmigen Teilchen, insbesondere die losen, festförmigen Partikel des körnige Gemenges, des in den Reaktor eingebrachten besagten Materials einen mittleren Durchmesser X_{50,3} (Volumenmittel) von 0,01 mm bis 5 cm, bevorzugt von 0,1 mm bis 5 cm, auf. Der mittlere Teilchengrößendurchmesser X_{50,3} wird durch Siebung oder mittels eines Partikelgrößenanalysators Camsizer der Fa. Retsch bestimmt. Nachfolgend werden abhängig von der Wahl des Reaktortyps weiter bevorzugte mittlere Teilchendurchmesser beschrieben.

Es hat sich als bevorzugt erwiesen, wenn das besagte Material vor der Einbringung in den Reaktor mit Blick auf seine Zusammensetzung entsprechend vorselektiert wird. Dadurch wird über die Betriebszeit der partiellen Oxidation eine verbesserte und konstantere Qualität des erhaltenen Produktgases erzielt. In diesem Zusammenhang erwies sich eine Mindestmenge an polymerer, organischer Verbindung, sowie ein Mindestanteil an Kohlenstoff als vorteilhaft.

Im Rahmen einer bevorzugten Ausführungsform ist es daher vorteilhaft, wenn die Gesamtmenge aller in dem Material enthaltenden polymeren, organischen Verbindungen mindestens 50 Gew.-%, bevorzugt von mindestens 60 Gew.-%, besonders bevorzugt von mindestens 75 Gew.-%, beträgt.

Eine weitere bevorzugte Ausführungsform des Verfahrens gekennzeichnet sich dadurch, dass die Gesamtmenge aller in dem Material enthaltenden polymeren, organischen Verbindungen einen Kohlenstoffanteil von mindestens 40,0 Gew.%, bevorzugt von mindestens 50 Gew.-%, aufweist.

Im Rahmen einer ganz besonders bevorzugten Ausführungsform des Verfahrens beträgt die Gesamtmenge aller in dem Material enthaltenden polymeren, organischen Verbindungen mindestens 50 Gew.-% (bevorzugt von mindestens 60 Gew.-%, besonders bevorzugt von mindestens 75 Gew.-%), wobei die Gesamtmenge aller in dem Material enthaltenden polymeren, organischen Verbindungen einen Kohlenstoffanteil von mindestens 40,0 Gew.%, bevorzugt von mindestens 50 Gew.-%, aufweist.

Ein besonders bevorzugtes und daher besonders geeignetes Material enthält als polymere, organische Verbindung solche, die ausgewählt werden aus mindestens einer homopolymeren oder copolymeren Verbindung aus der Gruppe Cellulose, Polyester, Polyamid, Polyurethan (PUR), Polyharnstoff, Polyisocyanurat (PIR), Polycarbonat, bevorzugt aus mindestens einer homopolymeren oder copolymeren Verbindung der Gruppe Polyurethan (PUR), Polyisocyanurat (PIR).

Polymere, organische Verbindungen, die mindestens eine Wiederholungseinheit aufweisen, die sich von Monomeren mit einer Carbonylgruppe ableiten, die in der polymeren, organischen Verbindung mindestens eine Verknüpfung, ausgewählt aus Urethanverknüpfung, Carbonatverknüpfung, Esterverknüpfung, Amidverknüpfung oder Isocyanuratverknüpfung bilden, sind ganz besonders bevorzugt als polymere, organische Verbindungen im besagten Material zur partiellen Oxidation gemäß einer weiteren bevorzugten Ausführungsform des erfindungsgemäßen Verfahrens geeignet. Eine besonders bevorzugte Ausführungsform des Verfahrens ist ein Verfahren, in welchem die Struktur der polymeren, organischen Verbindung des der partiellen Oxidation zugeführten Materials mindestens eine sich wiederholende Struktureinheit der Formel (I) enthält, 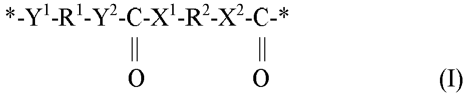 worin
X¹ und X² unabhängig voneinander eine direkte Bindung, O oder NH, bevorzugt O oder NH, bedeuten,
Y¹ und Y² unabhängig voneinander für O oder NH stehen,
R¹ und R² unabhängig voneinander jeweils für ein beliebiges bivalentes, Kohlenstoff enthaltendes Strukturelement stehen, und
eine mit ^{∗} gekennzeichnete kovalente Bindung eine Bindung zum Restmolekül der polymeren, organischen Verbindung bedeutet.

Im Rahmen dieser bevorzugtesten Ausführungsform erwies es sich als geeignet, wenn die Gesamtmenge der in dem Material enthaltenden polymeren, organischen Verbindungen mit mindestens einer sich wiederholenden Struktureinheit der Formel (I) mindestens 50 Gew.-%, bevorzugt von mindestens 60 Gew.-%, besonders bevorzugt von mindestens 75 Gew.-%, beträgt. Wenn gemäß Formel (I) X₁ und X₂ für eine Gruppe NH stehen und Y₁ und Y₂ für O stehen, dann enthält die polymere, organische Verbindung die erfindungsgemäß ganz besonders bevorzugte Urethanverknüpfung. Solche in dem besagten Material ganz besonders bevorzugt enthaltene polymere, organische Verbindungen können durch Umsetzung zumindest von
i1) mindestens einer organischen Isocyanatverbindung, enthaltend mindestens zwei bis vier, insbesondere zwei Isocyanatgruppen bindend an eine Kohlenwasserstoffeinheit mit 2 bis 8 Kohlenstoffatomen, bevorzugt mit 3 bis 8 Kohlenstoffatomen; mit
i2) mindestens einer organischen Verbindung mit mindestens zwei Hydroxygruppen,
erhalten werden.

Dabei kann die mindestens eine organische Isocyanatverbindung als besagte Kohlenwasserstoffeinheit eine Einheit enthalten, die die in i1) genannte Anzahl an Kohlenstoffatomen aufweist und sich ableitet von aliphatischen Kohlenwasserstoffeinheiten, cycloaliphatischen Kohlenwasserstoffeinheiten, araliphatischen Kohlenwasserstoffeinheiten, aromatischen Kohlenwasserstoffeinheiten oder heterozyklischen Kohlenwasserstoffeinheiten.

Als besagte organische Isocyanatverbindung wird besonders bevorzugt mindestens eine Verbindung gemäß Formel (II) ausgewählt

Q(NCO)ₙ (II)

worin n für eine Zahl von 2 bis 10, vorzugsweise von 2 bis 6, steht und Q für einen Rest steht, ausgewählt aus einem aliphatischen Kohlenwasserstoffrest mit 2 bis 70 Kohlenstoffatomen, (bevorzugt mit 3 bis 30 Kohlenstoffatomen), einem cycloaliphatischen Kohlenwasserstoffrest mit 3 bis 30 Kohlenstoffatomen, einem aromatischen Kohlenwasserstoffrest mit 6 bis 70 Kohlenstoffatomen (bevorzugt mit 6 bis 30 Kohlenstoffatomen) oder einem araliphatischen Kohlenwasserstoffrest mit 6 bis 70 Kohlenstoffatomen (bevorzugt mit 6 bis 30 Kohlenstoffatomen).

Dabei ist es bevorzugt, wenn Schritt i1) als mindestens eine organische Polyisocyanatverbindung mindestens ein Diisocyanat der Formel (IIIa) oder (IIIb) ausgewählt wird, 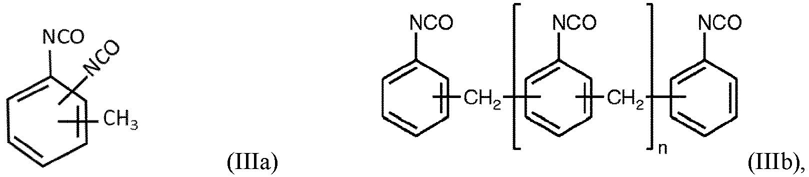 worin n für eine Zahl von 0 bis 8, insbesondere von 0 bis 4, weiter bevorzugt von 0 bis 2, steht.

Bevorzugt wird in Schritt i2) für die Herstellung der polymeren, organischen Verbindung mindestens eine organische Verbindung mit mindestens zwei Hydroxygruppen ausgewählt aus Polyesterpolyol, Polyetherpolyol, Polycarbonatpolyol, Polyetheresterpolyol, Polyacrylatpolyol Polyesterpolyacrylatpolyol oder Mischungen daraus, besonders bevorzugt ausgewählt aus der Gruppe der Polyetherpolyole und/oder der Polyesterpolyole.

Die OH-Zahl der eingesetzten organischen Verbindung mit mindestens zwei Hydroxygruppen oder der eingesetzten organischen Verbindungen mit mindestens zwei Hydroxygruppen, jeweils nach DIN 53240-1 (Juni 2013), beträgt bevorzugt von 15 bis 4000 mg KOH/g. Werden mehr als nur eine organische Verbindung mit mindestens zwei Hydroxygruppen verwendet, kann die Mischung aus besagten Verbindungen bevorzugt eine Hydroxylzahl zwischen 20 bis 200 mg KOH/g, insbesondere 25 bis 100 mg KOH/g aufweisen. Die OH-Zahl (auch: Hydroxylzahl) gibt im Falle einer einzelnen zugesetzten organischen Verbindung mit mindestens zwei Hydroxygruppen deren OH-Zahl an. Angaben der OH-Zahl für Mischungen beziehen sich auf die zahlenmittlere OH-Zahl der Mischung, berechnet aus den OH-Zahlen der einzelnen Komponenten in ihren jeweiligen molaren Anteilen. Die OH-Zahl gibt die Menge an Kaliumhydroxid in Milligramm an, welche der bei einer Acetylierung von einem Gramm Substanz gebundenen Menge Essigsäure gleichwertig ist. Sie wird im Rahmen der vorliegenden Ausführungen bestimmt nach der Norm DIN 53240-1 (Juni 2013).

Vorzugsweise weisen die organischen Verbindungen mit mindestens zwei Hydroxygruppen ein zahlenmittleres Molekulargewicht von ≥ 100 g/mol bis ≤ 15000 g/mol, insbesondere ≥ 2000 g/mol bis ≤ 12000 g/mol, bevorzugter ≥ 3500 g/mol bis ≤ 6500 g/mol auf.

Die zahlenmittlere Molmasse Mn (auch: Molekulargewicht) wird im Rahmen dieser Ausführungen durch Gelpermeationschromatographie nach DIN 55672-1 vom August 2007 bestimmt, falls an anderer Stelle nicht explizit anders festgelegt.

Die organischen Verbindungen mit mindestens zwei Hydroxylgruppen weisen eine Funktionalität von 1 bis 8 auf, wobei als "Funktionalität" im Rahmen der vorliegenden Erfindung die theoretische, aus den bekannten Einsatzstoffen und deren Mengenverhältnissen berechnete mittlere Funktionalität (Anzahl an gegenüber Isocyanaten bzw. gegenüber Polyolen reaktiven Funktionen im Molekül) bezeichnet wird.

Verwendbare Polyetheresterpolyole sind solche Verbindungen, die Ethergruppen, Estergruppen und OH-Gruppen enthalten. Vorzugsweise werden organische Dicarbonsäuren mit bis zu 12 Kohlenstoffatomen zur Herstellung der Polyetheresterpolyole eingesetzt, bevorzugt aliphatische Dicarbonsäuren mit 4 bis 6 Kohlenstoffatomen oder aromatische Dicarbonsäuren, die einzeln oder im Gemisch verwendet werden. Beispielhaft seien Korksäure, Azelainsäure, Decandicarbonsäure, Malonsäure, Phthalsäure, Pimelinsäure und Sebacinsäure sowie insbesondere Glutarsäure, Fumarsäure, Bernsteinsäure, Adipinsäure, Phthalsäure, Terephthalsäure und Isophthalsäure genannt. Neben organischen Dicarbonsäuren können auch Derivate dieser Säuren, beispielsweise deren Anhydride sowie deren Ester und Halbester mit niedermolekularen, monofunktionellen Alkoholen mit 1 bis 4 Kohlenstoffatomen eingesetzt werden. Der anteilige Einsatz der oben genannten biobasierten Ausgangsstoffe, insbesondere von Fettsäuren bzw. Fettsäurederivaten (Ölsäure, Sojaöl etc.) ist ebenfalls möglich und kann Vorteile aufweisen, z.B. im Hinblick auf Lagerstabilität der Polyolformulierung, Dimensionsstabilität, Brandverhalten und Druckfestigkeit der Schäume.

Als weitere Komponente zur Herstellung der Polyetheresterpolyole werden Polyetherpolyole eingesetzt, die man durch Alkoxylieren von Startermolekülen wie mehrwertigen Alkoholen erhält. Die Startermoleküle sind mindestens difunktionell, können aber gegebenenfalls auch Anteile höherfunktioneller, insbesondere trifunktioneller, Startermoleküle enthalten.

Startermoleküle sind zum Beispiel Ethylenglykol, Propylenglykol, 1,3-Butandiol, 1,4-Butandiol, 1,5-Pentendiol, 1,5-Pentandiol, 1,6-Hexandiol, 1,7-Heptandiol, 1,8-Octandiol, 1,10-Decandiol, 2-Methylpropan-1,3-diol, Neopentylglykol, 2,2-Dimethyl-1,3-propandiol, 3-Methyl-1,5-pentandiol, 2-Butyl-2-ethyl-1,3-propandiol, 2-Buten-1,4-diol und 2-Butin-1,4-diol, Diethylenglykol, Dipropylenglykol, Glycerin, Trimethylolpropan, di- und trifunktionelle Polyetherpolyole. Die Polyetherpolyole haben bevorzugt eine OH-Funktionalität von 2 bis 4 und ein Molekulargewicht Mn im Bereich von 62 bis 4500 g/mol und insbesondere ein Molekulargewicht Mn im Bereich von 62 bis 3000 g/mol. Auch Startermoleküle mit von OH verschiedenen Funktionalitäten können allein oder in Mischung eingesetzt werden.

Polyetheresterpolyole können auch durch die Alkoxylierung, insbesondere durch Ethoxylierung und/oder Propoxylierung, von Reaktionsprodukten, die durch die Umsetzung von organischen Dicarbonsäuren und deren Derivaten sowie Komponenten mit Zerewitinoff-aktiven Wasserstoffen, insbesondere Diolen und Polyolen, erhalten werden, hergestellt werden. Als Derivate dieser Säuren können beispielsweise deren Anhydride eingesetzt werden.

Die Polyesterpolyole können beispielsweise Polykondensate aus mehrwertigen Alkoholen, vorzugsweise Diolen, mit 2 bis 12 Kohlenstoffatomen, vorzugsweise mit 2 bis 6 Kohlenstoffatomen, und Polycarbonsäuren, wie z. B. Di-, Tri- oder sogar Tetracarbonsäuren oder Hydroxycarbonsäuren oder Lactonen sein, bevorzugt werden aromatische Dicarbonsäuren oder Gemische aus aromatischen und aliphatischen Dicarbonsäuren verwendet. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Als Carbonsäuren kommen insbesondere in Betracht: Bernsteinsäure, Glutarsäure, Adipinsäure, Korksäure, Azelainsäure, Sebazinsäure, Decandicarbonsäure, Tetrahydrophthalsäure, Hexahydrophthalsäure, Cyclohexandicarbonsäure, Tetrachlorphthalsäure, Itaconsäure, Malonsäure, 2-Methylbernsteinsäure, 3,3-Diethylglutarsäure, 2,2-Dimethylbernsteinsäure, Dodekandisäure, Endomethylentetrahydrophthalsäure, Dimerfettsäure, Trimerfettsäure, Zitronensäure, Trimellithsäure, Benzoesäure, Maleinsäure, Fumarsäure, Phthalsäure, Isophthalsäure und Terephthalsäure. Verwendet werden können ebenso Derivate dieser Carbonsäuren, wie beispielsweise Dimethylterephthalat. Die Carbonsäuren können dabei sowohl einzeln als auch im Gemisch verwendet werden. Als Carbonsäuren werden bevorzugt Adipinsäure, Sebacinsäure und/oder Bernsteinsäure, besonders bevorzugt Adipinsäure und/oder Bernsteinsäure, verwendet.

Hydroxycarbonsäuren, die als Reaktionsteilnehmer bei der Herstellung eines Polyesterpolyols mit endständigen Hydroxylgruppen mitverwendet werden können, sind beispielsweise Milchsäure, Äpfelsäure, Hydroxycapronsäure, Hydroxybuttersäure, Hydroxydecansäure, Hydroxystearinsäure und dergleichen. Geeignete Lactone sind unter anderem Caprolacton, Butyrolacton und Homologe.

Zur Herstellung der Polyesterpolyole kommen insbesondere auch biobasierte Ausgangsstoffe und/oder deren Derivate in Frage, wie z. B. Rizinusöl, Polyhydroxyfettsäuren, Ricinolsäure, Hydroxyl-modifizierte Öle, Weintraubenkernöl, schwarzem Kümmelöl, Kürbiskernöl, Borretschsamenöl, Sojabohnenöl, Weizensamenöl, Rapsöl, Sonnenblumenkernöl, Erdnussöl, Aprikosenkernöl, Pistazienöl, Mandelöl, Olivenöl, Macadamianussöl, Avocadoöl, Sanddornöl, Sesamöl, Hanföl, Haselnussöl, Primelöl, Wildrosenöl, Distelöl, Walnussöl, Fettsäuren, hydroxylmodifizierte und epoxidierte Fettsäuren und Fettsäureester, beispielsweise basierend auf Myristoleinsäure, Palmitoleinsäure, Ölsäure, Vaccensäure, Petroselinsäure, Gadoleinsäure, Erukasäure, Nervonsäure, Linolsäure, alpha- und gamma-Linolensäure, Stearidonsäure, Arachidonsäure, Timnodonsäure, Clupanodonsäure und Cervonsäure. Insbesondere bevorzugt sind Ester der Rizinolsäure mit mehrfunktionellen Alkoholen, z.B. Glycerin. Bevorzugt ist auch die Verwendung von Mischungen solcher biobasierten Säuren mit anderen Carbonsäuren, z.B. Phthalsäuren.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, 1,3-Butandiol, 1,4-Butandiol, 1,6-Hexandiol und Isomere, Neopentylglykol oder Hydroxypivalinsäureneopentylglykolester. Vorzugsweise verwendet werden Ethylenglykol, Diethylenglykol, 1,4-Butandiol, 1,5-Pentandiol, 1,6-Hexandiol oder Mischungen aus mindestens zwei der genannten Diole, insbesondere Mischungen aus 1,4-Butandiol, 1,5-Pentandiol und 1,6-Hexandiol.

Daneben können auch Polyole wie Trimethylolpropan, Glycerin, Erythrit, Pentaerythrit, Trimethylolbenzol oder Trishydroxyethylisocyanurat eingesetzt werden, wobei Glycerin und Trimethylolpropan bevorzugt sind.

Die als organische Verbindung mit mindestens zwei Hydroxygruppen verwendbaren Polyetherpolyole werden nach dem Fachmann bekannten Herstellungsmethoden erhalten, wie beispielsweise durch anionische Polymerisation von einem oder mehreren Alkylenoxiden mit 2 bis 4 Kohlenstoffatomen mit Alkalihydroxiden, wie Natrium- oder Kaliumhydroxid, Alkalialkoholaten, wie Natriummethylat, Natrium- oder Kaliumethylat oder Kaliumisopropylat, oder aminischen Alkoxylierungs-Katalysatoren, wie Dimethylethanolamin (DMEA), Imidazol und/oder Imidazolderivate, oder DMC-Katalysatoren unter Verwendung mindestens eines Startermoleküls, das 2 bis 8, vorzugsweise 2 bis 6 reaktive Wasserstoffatome gebunden enthält.

Geeignete Alkylenoxide sind beispielsweise Tetrahydrofuran, 1,3-Propylenoxid, 1,2- bzw. 2,3-Butylenoxid, Styroloxid und vorzugsweise Ethylenoxid und 1,2-Propylenoxid. Die Alkylenoxide können einzeln, alternierend nacheinander oder als Mischungen verwendet werden. Bevorzugte Alkylenoxide sind Propylenoxid und Ethylenoxid, besonders bevorzugt sind Copolymere des Propylenoxids mit Ethylenoxid. Die Alkylenoxide können in Kombination mit CO₂ umgesetzt werden.

Als Startermoleküle kommen beispielsweise in Betracht: Wasser, organische Dicarbonsäuren, wie Bernsteinsäure, Adipinsäure, Phthalsäure und Terephthalsäure, aliphatische und aromatische, gegebenenfalls N-mono-, N,N- und N,N'-dialkylsubstituierte Diamine mit 1 bis 4 Kohlenstoffatomen im Alkylrest, wie gegebenenfalls mono- und dialkylsubstituiertes Ethylendiamin, Diethylentriamin, Triethylentetramin, 1,3-Propylendiamin, 1,3- bzw. 1,4-Butylendiamin, 1,2-, 1,3-, 1,4-, 1,5- und 1,6-Hexamethylendiamin, Phenylendiamine, 2,3-, 2,4- und 2,6-Toluylendiamin und 2,2'-, 2,4'- und 4,4'-Diaminodiphenylmethan.

Vorzugsweise verwendet werden zwei oder mehrwertige Alkohole, wie Ethandiol, 1,2- und 1,3-Propandiol, Diethylenglykol, Dipropylenglykol, 1,4-Butandiol, 1,6-Hexandiol, Paraformaldehyd, Triethanolamin, Bisphenole, Glycerin, Trimethylolpropan, Pentaerythrit, Sorbit und Saccharose.

Verwendbare Polycarbonatpolyole sind Hydroxylgruppen aufweisende Polycarbonate, zum Beispiel Polycarbonatdiole. Diese entstehen in der Reaktion von Kohlensäurederivaten, wie Diphenylcarbonat, Dimethylcarbonat oder Phosgen, mit Polyolen, bevorzugt Diolen.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenole und lactonmodifizierte Diole der vorstehend genannten Art.

Statt oder zusätzlich zu reinen Polycarbonatdiolen können auch Polyether-Polycarbonatdiole eingesetzt werden, welche beispielsweise durch Copolymerisation von Alkylenoxiden, wie zum Beispiel Propylenoxid, mit CO₂ erhältlich sind.

Als Verbindungen mit gegenüber Isocyanaten reaktionsfähigen Wasserstoffatomen können auch Polymerpolyole, PHD-Polyole und PIPA-Polyole als organische Verbindung mit mindestens zwei Hydroxygruppen eingesetzt werden. Polymerpolyole sind Polyole, die Anteile von durch radikalische Polymerisation geeigneter Monomere wie Styrol oder Acrylnitril in einem Basispolyol erzeugten festen Polymeren enthalten. PHD (Polyhydrazodicarbonamid)-Polyole werden beispielsweise hergestellt durch in situ Polymerisation eines Isocyanats oder einer Isocyanat-Mischung mit einem Diamin und/oder Hydrazin (bzw. Hydrazinhydrat) in einem Polyol, bevorzugt einem Polyetherpolyol. Bevorzugt wird die PHD-Dispersion hergestellt durch Umsetzung einer Isocyanat-Mischung aus 75 bis 85 Gew.-% 2,4-Toluylendiisocyanat (2,4-TDI) und 15 bis 25 Gew.-% 2,6-Toluylendiisocyanat (2,6-TDI) mit einem Diamin und/oder Hydrazinhydrat in einem Polyetherpolyol hergestellt durch Alkoxylierung eines trifunktionellen Starters (wie beispielsweise Glycerin und/oder Trimethylolpropan). Bei PIPA-Polyolen handelt es sich um durch Polyisocyanat-Polyaddition mit Alkanolaminen-modifizierte Polyetherpolyole, wobei das Polyetherpolyol bevorzugt eine Funktionalität von 2,5 bis 4,0 und eine Hydroxylzahl von 3 mg KOH/g bis 112 mg KOH/g (Molekulargewicht 500 g/mol bis 18000 g/mol) aufweist.

Es können auch Isocyanat-reaktive Substanzen mit zellöffnender Wirkung eingesetzt werden, wie beispielsweise Copolymere aus Ethylenoxid und Propylenoxid mit einem Überschuss an Ethylenoxid oder aromatischen Diaminen wie Diethyltoluendiamin.

Für die Herstellung von Polyurethanschaumstoffen im Kaltschaumverfahren werden im Rahmen einer weiteren Ausführungsform mindestens zwei Hydroxylgruppen aufweisender Polyether mit einer OH-Zahl von 20 bis 50 mg KOH/g als organische Verbindung mit mindestens zwei Hydroxygruppen eingesetzt, wobei die OH-Gruppen zu mindestens 80 Mol-% aus primärem OH-Gruppen bestehen (Bestimmung mittels 1H-NMR (z.B. Bruker DPX 400, Deuterochloroform)). Besonders bevorzugt beträgt die OH-Zahl 25 bis 40 mg KOH/g, ganz besonders bevorzugt 25 bis 35 mg KOH/g.

Neben der zuvor definierten Struktureinheit der Formel (I) kann die polymere, organische Verbindung des Materials Isocyanurat-Struktureinheiten der Formel 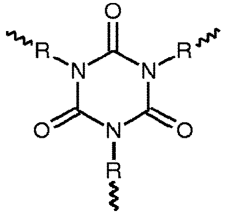 enthalten, worin R für einen bivalenten Kohlenwasserstoffrest, insbesondere für einen bivalenten aromatischen Kohlenwasserstoffrest, steht. Entsprechende Isocyanurat-Struktureinheiten finden sie beispielsweise zu einem großen Teil in Polyurethanhartschäumen.

Bevorzugt handelt es sich bei dem die besagte polymere, organische Verbindung enthaltenden Material um einen Schaum, noch bevorzugter um einen Polyurethanschaum. Wenn das Material in Form eines Polyurethanschaumes vorliegt, handelt es sich wiederum bevorzugt um einen Polyurethanweichschaum oder einen Polyurethanhartschaum.

Wenn gemäß Formel (I) X₁ und X₂ für eine Gruppe O stehen und Y₁ und Y₂ für O stehen, dann enthält die polymere, organische Verbindung die erfindungsgemäß ganz besonders bevorzugte Carbonatverknüpfung.

Für das erfindungsgemäße Verfahren geeignete polymere organische Verbindungen dieser Ausführungsform sind beispielsweise aromatische Polycarbonate und/oder aromatische Polyestercarbonate. Diese sind literaturbekannt oder nach literaturbekannten Verfahren herstellbar (zur Herstellung aromatischer Polycarbonate siehe beispielsweise Schnell, "Chemistry and Physics of Polycarbonates", Interscience Publishers, 1964 sowie die DE-AS 1 495 626, DE-A 2 232 877, DE-A 2 703 376, DE-A 2 714 544, DE-A 3 000 610, DE-A 3 832 396; zur Herstellung aromatischer Polyestercarbonate, z. B. DE-A 3 007 934).

Die Herstellung der als eine entsprechende polymere, organische Verbindung einsetzbaren aromatischen Polycarbonate erfolgt z. B. durch Umsetzung von Verbindungen mit mindestens zwei Hydroxygruppen, insbesondere von Diphenolen, mit Kohlensäurehalogeniden, vorzugsweise Phosgen und/oder mit aromatischen Dicarbonsäuredihalogeniden, vorzugsweise Benzoldicarbonsäuredihalogeniden, nach dem Phasengrenzflächenverfahren, gegebenenfalls unter Verwendung von Kettenabbrechern, beispielsweise Monophenolen und gegebenenfalls unter Verwendung von trifunktionellen oder mehr als trifunktionellen Verzweigern, beispielsweise Triphenolen oder Tetraphenolen. Ebenso ist eine Herstellung über ein Schmelzepolymerisationsverfahren durch Umsetzung von Verbindungen mit mindestens zwei Hydroxylgruppen, insbesondere von Diphenolen, mit beispielsweise Diphenylcarbonat möglich.

Erfindungsgemäß sind solche besagte Materialien vorteilhaft einsetzbar, in denen die polymere, organische Verbindung mindestens eine Verbindung ist, die durch Umsetzung von zumindest den Verbindungen (i) mindestens einer aromatischen Verbindung mit mindestens zwei Hydroxylgruppen, besonders bevorzugt Bisphenol-A, und (ii) Phosgen oder Diphenylcarbonat erhalten wurde.

Zur Darstellung der Polycarbonat enthaltenden Verbindung wird bevorzugt mindestens eine aromatische Verbindung mit mindestens zwei Hydroxylgruppen verwendet, die aus der allgemeinen Formel (IV) ausgewählt wird, 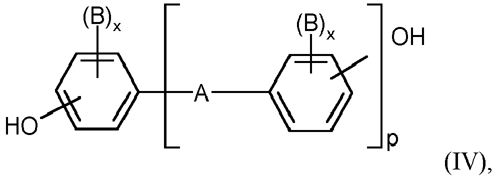 worin
A eine Einfachbindung, Ci bis Cs-Alkylen, C₂ bis Cs-Alkyliden, C₅ bis C₆-Cycloalkyliden, - O-, -SO-, -CO-, -S-, -SO₂-, C₆ bis C₁₂-Arylen, an das weitere aromatische gegebenenfalls Heteroatome enthaltende Ringe kondensiert sein können,
   oder ein Rest der Formel (V) oder (VI) 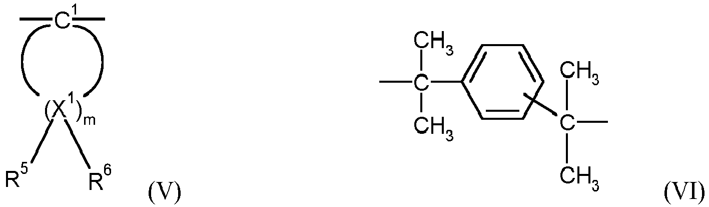
B jeweils Ci bis C₁₂-Alkyl, vorzugsweise Methyl, Halogen, vorzugsweise Chlor und/oder Brom
x jeweils unabhängig voneinander 0, 1 oder 2,
p 1 oder 0 sind, und
R⁵ und R⁶ für jedes X¹ individuell wählbar, unabhängig voneinander Wasserstoff oder Ci bis C₆-Alkyl, vorzugsweise Wasserstoff, Methyl oder Ethyl,
X¹ Kohlenstoff und
m eine ganze Zahl von 4 bis 7, bevorzugt 4 oder 5 bedeuten, mit der Maßgabe, dass an mindestens einem Atom X¹, R⁵ und R⁶ gleichzeitig Alkyl sind.

Bevorzugte aromatische Verbindungen mit mindestens zwei Hydroxylgruppen sind Hydrochinon, Resorcin, Dihydroxydiphenole, Bis-(hydroxyphenyl)-C₁-C₅-alkane, Bis-(hydroxyphenyl)-C₅-C₆-cycloalkane, Bis-(hydroxyphenyl)-ether, Bis-(hydroxyphenyl)-sulfoxide, Bis-(hydroxyphenyl)-ketone, Bis-(hydroxyphenyl)-sulfone und α,α-Bis-(hydroxyphenyl)-diisopropyl-benzole sowie deren kernbromierte und/oder kernchlorierte Derivate.

Besonders bevorzugte aromatische Verbindungen mit mindestens zwei Hydroxylgruppen sind 4,4'-Dihydroxydiphenyl, Bisphenol-A, 2,4-Bis(4-hydroxyphenyl)-2-methylbutan, 1,1-Bis-(4-hydroxyphenyl)-cyclohexan, 1,1-Bis-(4-hydroxyphenyl)-3.3.5-trimethylcyclohexan, 4,4'-Dihydroxydiphenylsulfid, 4,4'-Dihydroxydiphenylsulfon sowie deren di- und tetrabromierten oder chlorierten Derivate wie beispielsweise 2,2-Bis(3-Chlor-4-hydroxyphenyl)-propan, 2,2-Bis-(3,5-dichlor-4-hydroxyphenyl)-propan oder 2,2-Bis-(3,5-dibrom-4-hydroxyphenyl)-propan. Insbesondere bevorzugt ist 2,2-Bis-(4-hydroxyphenyl)-propan (Bisphenol-A).

Es können die aromatischen Verbindungen mit mindestens zwei Hydroxylgruppen einzeln oder als beliebige Mischungen eingesetzt werden. Die aromatischen Verbindungen mit mindestens zwei Hydroxylgruppen sind literaturbekannt oder nach literaturbekannten Verfahren erhältlich.

Einsetzbare thermoplastische, aromatische Polycarbonate haben im Rahmen einer weiteren Ausführungsform der Erfindung mittlere Molekulargewichte (Gewichtsmittel M_{w}, gemessen durch GPC (Gelpermeationschromatographie) mit Polycarbonatstandard auf Basis Bisphenol A) von bevorzugt 20000 bis 40000 g/mol, weiter bevorzugt 24000 bis 32000 g/mol, besonders bevorzugt 26000 bis 30000 g/mol.

Die thermoplastischen, aromatischen Polycarbonate können in bekannter Weise verzweigt sein, und zwar vorzugsweise durch den Einbau von 0,05 bis 2,0 Mol%, bezogen auf die Summe der eingesetzten aromatische Verbindung mit mindestens zwei Hydroxylgruppen, an dreifunktionellen oder mehr als dreifunktionellen Verbindungen, beispielsweise solchen mit drei und mehr phenolischen Gruppen.

Bevorzugt werden lineare Polycarbonate, weiter bevorzugt auf Basis von Bisphenol-A, eingesetzt.

Geeignet als polymere, organische Verbindung sind sowohl Homopolycarbonate als auch Copolycarbonate. Zur Herstellung erfindungsgemäß bevorzugt einsetzbarer Copolycarbonate können auch 1 bis 25 Gew.%, vorzugsweise 2,5 bis 25 Gew.%, bezogen auf die Gesamtmenge an einzusetzender aromatischer Verbindung mit mindestens zwei Hydroxylgruppen, Polydiorganosiloxane mit Hydroxyaryloxy-Endgruppen eingesetzt werden. Diese sind bekannt (US 3 419 634) und nach literaturbekannten Verfahren herstellbar. Ebenfalls geeignet sind Polydiorganosiloxanhaltige Copolycarbonate; die Herstellung der Polydiorganosiloxanhaltiger Copolycarbonate ist beispielsweise in der DE-A 3 334 782 beschrieben.

Aromatische Dicarbonsäuredihalogenide zur Herstellung von aromatischen Polyestercarbonaten sind vorzugsweise die Disäuredichloride der Isophthalsäure, Terephthalsäure, Diphenylether-4,4'-dicarbonsäure und der Naphthalin-2,6-dicarbonsäure.

Die aromatischen Polyestercarbonate können sowohl linear als auch in bekannter Weise verzweigt sein (siehe dazu DE-A 2 940 024 und DE-A 3 007 934), wobei lineare Polyestercabonate bevorzugt sind.

Die partielle Oxidation des eingesetzten Materials wird in dem Reaktor bei einer Temperatur von mindestens 400°C durchgeführt. Vorzugsweise wird die partielle Oxidation des Materials in dem Reaktor bei einer Temperatur in einem Temperaturbereich von 600 °C bis 1500°C, insbesondere von 850°C bis 1400°C, weiter bevorzugt von 1100°C bis 1300°C, durchgeführt.

Die partielle Oxidation des eingesetzten Materials wird im Rahmen einer bevorzugten Ausführungsform bei einem absoluten Druck von mehr als 1 bar, bevorzugt bei einem absoluten Druck in einem Bereich von 2 bis 80 bar, besonders bevorzugt bei einem absoluten Druck in einem Bereich von 2 bis 50 bar, durchgeführt.

Die partielle Oxidation des Materials kann in mindestens einem der folgenden drei Reaktoren ausgeführt werden: Reaktor für Flugstromvergasung, Reaktor für Wirbelschichtvergasung und Reaktor für Festbettvergasung.

Für die Verwendung in einem Reaktor für Flugstromvergasung müssen die polymeren, organischen Verbindungen, bzw. das diese enthaltende Material, auf eine Korngröße mit einem mittleren Teilchendurchmesser X_{50,3} von < 0,1 mm (Staub) gemahlen werden. Die Zufuhr in den Reaktor erfolgt entweder pneumatisch oder als Aufschlämmung. Die größte Einschränkung dieser Art der partiellen Oxidation für die chemische Wiederverwertung von Abfall ist die Mahlbarkeit und Förderbarkeit von Ausgangsstoffen aus heterogenem Abfall. Eine thermische Behandlung von Biomasse (Torrefaktion) bei 200 - 300 °C unter O₂ Ausschluss wird zur Herstellung einer "Biokohle" mit ähnlicher Mahlbarkeit wie Steinkohle verwendet. In einer anderen Variante kann eine vorgeschaltete Pyrolyse zur Herstellung eines pumpbaren Pyrolyseöls verwendet werden. Das durch Abfall-Pyrolyse gewonnene Öl kann entweder direkt oder in Form einer mit dem festen Pyrolyserückstand (Pyrolysekoks) vermischten Aufschlämmung partiell oxidiert werden. Diese Prozesskonfiguration wurde von der Firma Noel (Noell-Konversionsverfahren zur Verwertung und Entsorgung von Abfällen, Jürgen Carl EF-Verl. für Energie- und Umwelttechnik, 1994. ISBN: 3924511829) entworfen.

Eine andere Variante stellt die Nutzung eines Reaktors für Wirbelschichtvergasung dar. Die Vergasung von Abfall in Wirbelschichtreaktoren ist vielfach mit den Technologien EBARA (Showa Denko, Japan), ENERKEM (Enerkem, Edmonton, Kanada) und der Großdemonstration der Hochtemperatur-Winkler-Gasförderung (HTW) durch die Rheinbraun AG (heute RWE) von 1993 bis 1997 in Berrenrath, Deutschland, bekannt. Die Vorbehandlung zur Einspeisung der polymeren, organischen Verbindungen, bzw. des diese enthaltenden Materials in den Reaktor erfordert eine Zerkleinerung auf mit einem mittleren Teilchendurchmesser X_{50,3} von 30 - 80 mm. Die Einspeisung in den Reaktorbehälter erfolgt über Schneckenförderer begrenzt den Vergaserdruck auf maximal 10 bar. Die Technologien von ENERKEM und EBARA ermöglichen die Vergasung von hochkalorischem Abfall (Kunststoffabfall oder Kunststoffreiche Ersatzbrennstoffe. Wirbelschichtvergaser werden bei milden Temperaturen von 700 - 950 °C deutlich unterhalb des Asche-Schmelzpunkts des Einsatzmaterials betrieben, um Anbackungen und Agglomerationen im Reaktor zu vermeiden. Ein weiterer Vorteil dieser milden Reaktortemperatur ist der unvollständige Kohlenstoffumsatz in der Wirbelschicht. Weiterhin enthält das Rohgas aus Reaktoren zur Wirbelschichtvergasern typischerweise erhebliche Mengen an Methan und anderen Kohlenwasserstoffen. Um dies zu kompensieren und eine hohe syn-Gasausbeute von H₂ und CO zu gewährleisten, nutzen die Prozesse von ENERKEM und EBARA eine zweite Hochtemperatur-Stufe zur partiellen Oxidation (ca. 1400 °C), die direkt hinter dem Wirbelbett angeordnet ist, um Flugasche zu schmelzen und Kohlenwasserstoffe im Produktgas der ersten Stufe zum finalen Kohlenmonoxid-haltigen Produktgasstrom umzuwandeln. ENERKEM nennt diese zweite Stufe "Thermoreformer", EBARA bezeichnet einen "Hochtemperatur-Vergasungsofen". Die hohe Temperatur dieser zweiten partiellen Oxidationsstufe erhöht die CO₂-Produktion.

Im Rahmen einer weiteren Ausführungsform des erfindungsgemäßen Verfahrens wird die partielle Oxidation des Materials bevorzugt in mindestens zwei Schritten durchgeführt, wobei in einem ersten Schritt eine partielle Oxidation des Materials in einem ersten Reaktor bei einer Temperatur in einem Temperaturbereich von 400 °C bis 800°C durchgeführt wird, das Produktgas des ersten Reaktors in einen zweiten Reaktor überführt wird und dort in einem weiteren Schritt unter Zuführung eines sauerstoffhaltigen Gasstromes einer partiellen Oxidation bei einer Temperatur von mehr als 800 °C, bevorzugt bei einer Temperatur in einem Bereich von 1300°C bis 1500°C, unterworfen wird unter Erhalt des Kohlenmonoxid-haltigen Produktgas-Stroms.

Die Festbettvergasung unterscheidet sich zwischen Prozessen mit Trockenasche und Entladung mit Flüssigschlacke. Das British Gas-Lurgi (BGL)-Verfahren (British Gas/Lurgi slagging gasifier, Veröffentlicht am: 1993-08-30) wurde erfolgreich für die Co-Gasifikation von bis zu 80 % Abfall (Ersatzbrennstoffe, modernisierter kommunaler fester Abfall und industrieller Abfall, Shredder-Leichtfraktion, Mischkunststoff-Abfall, Abfall, Abwasser-Schlamm) mit Braunkohle und Steinkohle angewendet.

Die Vorbehandlung von zur partiellen Oxidation bestimmtem Abfall in Festbettvergasern mit Schlacken erfordert eine Granulierung, Verdichtung oder Pressen. Das Abfall-Einsatzmaterial wird dann über ein Schleusensystem am Kopf des Reaktors (Vergasers) in den Reaktor eingeleitet. So kann der Reaktor bei Drücken bis zu 40 bar betrieben werden, wodurch der Verdichter für die nachfolgende Gasbehandlung und chemische Synthese reduziert wird. Die Rohre erwärmen in der Nähe des Reaktorbodens das Einsatzmaterial deutlich über dem Asche-Schmelzpunkt und bilden ein Schlackenbad. Verglaste, nahezu kohlenstofffreie Schlacke (< 1 Gew.-%) wird diskontinuierlich in eine wassergefüllte Kammer ausgetragen. Das Produktgas bewegt sich durch den Reaktor (Vergaser) nach oben und durchläuft eine Nachvergasungszone als zweiten Schritt der partiellen Oxidation, in der zusätzlicher Sauerstoff und bevorzugt zusätzlich gasförmiges Wasser (Dampf) eingeleitet wird, um die Temperatur auf 1000 - 1150 °C zu erhöhen und Methan und Kohlenwasserstoffe umzuwandeln, bevor es als Kohlenmonoxid-haltiger Produktgasstrom den Reaktor verlässt.

Das aus dem erfindungsgemäßen Verfahren erhältliche Kohlenmonoxid-haltige Produktgas der partiellen Oxidation enthält neben Kohlenmonoxid meistens Anteile von CO₂, sowie von Wasser, Wasserstoffgas und Methan. Weiterhin sind in dem aus dem erfindungsgemäßen Verfahren erhältlichen Kohlenmonoxid-haltigen Produktgas der partiellen Oxidation zusätzlich partikelförmige Feststoffe dispergiert.

Der Kohlenmonoxid-haltige Produktgas-Strom wird daher anschließend einer Reinigung zugeführt, in der in Rangfolge zunächst der Kohlenmonoxid-haltige Produktgas-Strom zur Abtrennung von Feststoff einem Waschschritt zugeführt wird, worin
(i) Wasser mit dem Kohlenmonoxid-haltigen Produktgas-Strom in Kontakt gebracht wird, wodurch der im Kohlenmonoxid-haltigen Produktgas-Strom dispergierte, partikelförmige Feststoff eine Schlacke bildet und diese Schlacke entfernt und abgeführt wird,
(ii) der von partikelförmigem Feststoff gereinigte, Kohlenmonoxid-haltige Produktgas-Strom ausgebracht wird.

In dem Waschschritt kann das der partiellen Oxidation entnommene Kohlenmonoxid-haltige Produktgas zunächst mit einer Temperatur von mehr als 400°C in den Waschschritt eingebracht werden. In diesem Falle wird das besagte Produktgas nach Inkontaktbringen mit Wasser abgekühlt und das dabei verdampfende Wasser, beispielsweise als Kondensat, abgetrennt und gegebenenfalls in den Prozess zurückgeführt.

Der aus dem Waschschritt ausgebrachte, von partikelförmigem Feststoff gereinigte, Kohlenmonoxid-haltige Produktgas-Strom hat bevorzugt eine Temperatur von höchstens 100 °C besonders bevorzugt von höchstens 80°C.

Es hat sich als ganz besonders bevorzugt herausgestellt, wenn für das Inkontaktbringen das Wasser beim Waschschritt in den Kohlenmonoxid-haltigen Produktgas-Strom gesprüht wird.

Von partikelförmigem Feststoff gereinigtes, Kohlenmonoxid-haltiges Produktgas wird, gegebenenfalls nach Ausführung weiterer optionaler von Schritt c1) bis c5) verschiedener Reinigungsschritte, gemäß Rangfolge c2) als nächstes in einem Trockenschritt zumindest einer Abtrennung von Wasser zugeführt, Wasser abgetrennt und der resultierende Kohlenmonoxid-haltige Produktgas-Strom ausgebracht.

In einer Wasser-Abtrenneinheit wird zur Abtrennung des Wassers beispielsweise das der partiellen Oxidation entnommene Kohlenmonoxid-haltige Produktgas abgekühlt und das Wasser, beispielsweise als Kondensat, abgetrennt.

Das abgetrennte Wasser kann dem Waschschritt c1) zugeführt werden.

Ein mittels Abtrennung von Wasser gereinigter Kohlenmonoxid-haltiger Produktgas-Strom wird, gegebenenfalls nach Ausführung weiterer optionaler von Schritt c1) bis c5) verschiedener Reinigungsschritte, gemäß Rangfolge c3) als nächstes zumindest einer Abtrennung von Kohlendioxid zugeführt, Kohlendioxid abgetrennt und der resultierende Kohlenmonoxid-haltige Produktgas-Strom und Kohlendioxid ausgebracht. Dabei kann das ausgebrachte Kohlendioxid an die Umwelt abgegeben werden oder wie später beschrieben zusätzlich zum sauerstoffhaltigen Gas in die partielle Oxidation eingebracht und dort verwertet werden.

Die Abtrennung von CO₂, kann als "Aminwäsche" ausgeführt werden, wobei das Kohlenmonoxid-haltige Produktgas hier insbesondere die grundsätzlich bekannte Wäsche des Gasgemisches nach dem Prinzip der Chemisorption mit Aminen, wie Monoethanolamin (MEA) Diethanolamin (DEA), Methyldiethanolamin (MDEA) oder Diglycolamin (DGA) durchläuft, welche in einer Absorptionskolonne eine hohe Reinheit des gereinigten Gasgemisches erreicht.

Zur Verringerung des CO₂-Fußabdrucks ist es im Rahmen einer weiteren Ausführungsform der vorliegenden Erfindung ganz besonders bevorzugt, wenn das in Schritt c3) ausgebrachte, abgetrennte Kohlendioxid zu einem Reaktor der partiellen Oxidation zurückgeführt und in diesen Reaktor eingebracht wird.

Vorzugsweise wird im Rahmen einer Ausführungsform der vorgenannten CO₂-Rückführung das Kohlendioxid in einer Menge von höchstens 1 kg pro kg polymerer, organischer Verbindung, bevorzugt von höchstens 0,9 kg pro kg polymerer, organischer Verbindung, besonders bevorzugt von 0,8 kg pro kg polymerer, organischer Verbindung, der partiellen Oxidation zugefügt.

Wird die partielle Oxidation des Materials in mindestens zwei Reaktoren durchgeführt, wird das in Schritt c3) ausgebrachte, abgetrennte Kohlendioxid zumindest zum ersten Reaktor der partiellen Oxidation zurückgeführt und in diesen Reaktor eingebracht.

Wenngleich die CO₂-Rückführung besonders bevorzugt ist, kann eine Zuführung von CO₂ zur partiellen Oxidation ebenfalls aus einer weiteren CO₂-Quelle erfolgen, beispielsweise das bei der Bereitstellung von Wärmeenergie emittierte CO₂ aus der Brennkammer eines Heizelementes, oder CO₂ aus einer externen CO₂-Quelle. Dies kann auch zusätzlich zur vorgenannten CO₂-Rückführung erfolgen.

Als eine weitere CO₂-Quelle dient bevorzugt mindestens eine externe CO₂-Quelle, die CO₂ beiträgt, welches nicht durch das erfindungsgemäße Verfahren emittiert wird. Eine externe CO₂ Quelle wäre z.B. das CO₂, das bei einer Zementherstellung, bei einer H₂-Herstellung für die Ammoniaksynthese anfällt, bei Verbrennung von Brennstoffen (z.B. einer Abfallverbrennung) im Abgas entsteht, oder CO₂, das aus der Luft gewonnenen wird. Dieses CO₂ aus einer externen CO₂-Quelle wird im Rahmen einer bevorzugten Ausführungsform des erfindungsgemäßen Verfahrens durch Absorption eines CO₂ -Anteils aus (i) Prozessgasen oder Abgasen, die aus mindestens einem Prozess ausgewählt aus Zementherstellung, H2-Herstellung, Verbrennung ausgewählt wird und/oder (ii) aus Luft durch einleiten in Alkalilauge, zum Beispiel Kalilauge, erfolgen. Hierbei bildet sich Kaliumhydrogencarbonat, welches anschließend wieder zu CO₂ und Kalilauge thermisch zersetzt werden kann. Das dabei freigesetzte CO₂ wird dann der partiellen Oxidation zur Synthese von Kohlenmonoxid zugeführt. Zur Bereitstellung der für die CO₂ Freisetzung notwendigen Wärmeenergie kann beispielsweise die erzeugte Wärmeenergie aus der partiellen Oxidation eingesetzt werden.

Optional können weitere Reinigungsschritte des CO₂ durchgeführt werden, um eine ausreichende Reinheit des CO₂ für die besagte partielle Oxidation zu erzeugen.

Ein mittels Abtrennung von Kohlendioxid gereinigter Kohlenmonoxid-haltiger Produktgas-Strom wird, gegebenenfalls nach Ausführung weiterer optionaler von Schritt c1) bis c5) verschiedener Reinigungsschritte, gemäß Rangfolge als nächstes zumindest einer Trenneinheit zur Abtrennung von Kohlenmonoxid c4) zugeführt, eine Abtrennung von Kohlenmonoxid durchgeführt und das resultierende Kohlenmonoxid und ein Wasserstoffgas-haltiges Restgas ausgebracht.

Dabei bildet sich bevorzugt zumindest ein Gasstrom, dessen Gas bei 25°C und 1013 mbar mindestens 95 Vol.% Kohlenmonoxid, bevorzugter mindestens 99 Vol.% Kohlenmonoxid, enthält, der als Kohlenmonoxid ausgebracht wird. Der besagte, von Kohlendioxid gereinigte Kohlenmonoxid-haltige Produktgas-Strom wird in der Trenneinheit zur Abtrennung von Kohlenmonoxid (nachfolgend auch als H₂-CO Trenneinheit bezeichnet) vorzugsweise zunächst in zwei Gasströme aufgetrennt. Es entsteht dabei ein Gas in Form eines Gasstroms, das mindestens 95 Vol.% (bevorzugt mindestens 99 Gew.-%) Kohlenmonoxid enthält, ein weiteres Gas in Form eines Gasstroms, dessen größter Bestandteil Wasserstoff ist und unter anderem Kohlenmonoxid und Methan enthält. Das weitere Gas wird auch als Restgas der H₂-CO Trennung oder falls keine Restgasbehandlung erfolgt, als Endgas bezeichnet. Eine nach diesem Prinzip der Auftrennung arbeitende H₂-CO Trenneinheit ist die sogenannte Coldbox.

Das Wasserstoff-haltige Restgas der H₂-CO Trennung kann zur Aufkonzentrierung des Wasserstoffs einer anschließenden Restgasbehandlung ausgesetzt werden. Dabei wird ein mittels besagter Trenneinheit zur Abtrennung von Kohlenmonoxid abgetrenntes, Wasserstoffgas-haltiges Restgas einer Restgasbehandlung zugeführt, Wasserstoffgas abgetrennt und Wasserstoffgas und ein Endgas ausgebracht. Nach der Aufarbeitung durch die Restgasbehandlung wird ein mit Wasserstoffgas angereichertes Gas in Form eines Gasstroms und ein weiteres Gas - das sogenannte Restgas der Restgasbehandlung oder Endgas - in Form eines Gasstroms, erhalten, das eine Mischung von CO, Methan und einer geringeren Menge Wasserstoff enthält, als das mit Wasserstoffgas angereicherte Gas.

Das aus der Abtrennung von Kohlenmonoxid abgetrennte Wasserstoffgas des erfindungsgemäßen Verfahrens kann für die Hydrierung von Nitroverbindungen zur Herstellung von Aminen als Rohstoff für die Isocyanatsynthese eingesetzt werden. Im Rahmen einer bevorzugten Ausführungsform des Verfahrens wird nach der Abtrennung von Wasserstoff in der H₂-CO-Trennung, insbesondere unter Verwendung einer Coldbox, der abgetrennte gasförmige Wasserstoff aus dem Restgas der H₂-CO Trennung nach weiterer Restgasbehandlung als daraus hervorgehender Wasserstoff in eine Hydrierung von organischer Nitroverbindung zur Herstellung von organischem Amin als Rohstoff für organisches Isocyanat eingespeist.

Wie erwähnt erfolgt im Rahmen einer bevorzugten Ausführungsform des Verfahrens zusätzlich gemäß Schritt d) eine Phosgensynthese durch Umsetzung von zumindest Chlorgas und Kohlenmonoxid, wobei als Kohlenmonoxid mindestens das Kohlenmonoxid aus dem Reinigungsschritt c) umgesetzt und Phosgen ausgebracht wird. Insbesondere wird dabei bevorzugt die besagte Reinigung gemäß für einen hohen Reinheitsgrad geeigneten Ausführungsform durchgeführt und das verbliebene Kohlenmonoxid aus der Reinigung in die Phosgensynthese eingespeist.

Für die Bereitstellung des Chlors für die im Rahmen dieser Ausführungsform erfolgende Phosgensynthese ist dem Fachmann die Herstellung von Chlorgas aus elektrochemischer Oxidation nach der Salzsäure-Elektrolyse mit Gasdiffusionselektrode (auch als HCl ODC Elektrolyse-Verfahren bezeichnet (ODC steht für Oxygen Depleting Electrode, als eine ODC wird beispielsweise die sogenannte SVK (Sauerstoffverzehrkathode) eingesetzt); geeignete Elektrolysezelle vgl. US,6022,634 A, WO 03/31690 A1), die Herstellung von Chlorgas aus Salzsäure-Diaphragmaelektrolyse (vgl. EP 1 103 636 A1), die Herstellung von Chlorgas aus thermokatalytischer Gasphasenoxidation (vgl. WO 2012/025483 A2), sowie die Herstellung von Chlor aus Chloralkali-Elektrolyse (vgl. WO 2009/007366 A2) hinlänglich bekannt. Auf den Inhalt der vorgenannten, im Zusammenhang mit der Herstellung von Chlorgas zitierten Schriften wird ausdrücklich und vollumfänglich Bezug genommen. Das für die Synthese von Phosgen benötigte Chlor wird in einer bevorzugten Variante dieser Ausführungsform des Verfahrens elektrolytisch hergestellt, insbesondere durch elektrochemische Oxidation nach der Salzsäure-Elektrolyse mit Gasdiffusionselektrode, durch elektrochemische Oxidation nach der Salzsäure-Diaphragmaelektrolyse oder durch elektrochemische Oxidation nach der Chloralkali-Elektrolyse. Dabei ist es wiederum besonders bevorzugt, wenn die besagte elektrochemische Oxidation jeweils unter Verwendung von aus regenerativer Energie erzeugtem elektrischem Strom durchgeführt wird, insbesondere aus regenerativer Energie in Form von Windkraft, Sonnenenergie oder Wasserkraft.

Das durch die Phosgensynthese gebildete Phosgen wird im Rahmen einer besonders bevorzugten Ausführungsform des Verfahrens in einem weiteren Schritt e) zur Herstellung von organischem Isocyanat (9) eingesetzt, worin das Phosgen (8a) aus der Phosgensynthese (8) mit mindestens einer organischen Aminoverbindung (20a) umgesetzt und zumindest organisches Isocyanat (9a) ausgebracht wird. Vorzugsweise werden zumindest organisches Isocyanat und Chlorwasserstoff ausgebracht.

Es sind generell solche erfindungsgemäßen Verfahren dieser Ausführungsform bevorzugt, in denen das gewonnene organische Isocyanat mindestens zwei Isocyanatgruppen enthält. Zu diesem Zweck wird bei der Synthese wiederum bevorzugt als Reaktand organisches Amin mit mindestens zwei Aminogruppen eingesetzt.

Besonders bevorzugt enthält das gewonnene organische Isocyanat mindestens zwei Isocyanatgruppen und weist eine Molmasse von höchstens 1000 g/mol, insbesondere von höchstens 800 g/mol, auf.

Ganz besonders bevorzugt eingesetzte organische Amine werden ausgewählt aus Toluylendiamin (TDA), Methylendi(phenylamin) (MDA) (wiederum bevorzugt ausgewählt aus Diphenylmethan-2,2'-diamin, Diphenylmethan-2,4'-diamin, Diphenylmethan-4,4'-diamin oder Mischungen daraus), Hexamethylendiamin, Isophorondiamin, 1,3-Bis(aminomethyl)benzol, Cyclohexyldiamin oder Mischungen daraus, wobei TDA, MDA oder Mischungen daraus ganz besonders bevorzugte organische Isocyanate sind.

Ganz besonders bevorzugt erhaltene organische Isocyanate werden ausgewählt aus Toluylendiisocyanat (TDI), Methylendi(phenylisocyanat) (MDI) (wiederum bevorzugt ausgewählt aus Diphenylmethan-2,2'-diisocyanat, Diphenylmethan-2,4'-diisocyanat, Diphenylmethan-4,4'-diisocyanat oder Mischungen daraus), Hexamethylendiisocyanat (HDI), Isophorondiisocyanat (IPDI), 1,3-Bis(isocyanatomethyl)benzol (XDI), Cyclohexyldiisocyanat (CHDI) oder Mischungen daraus, wobei TDI, MDI oder Mischungen daraus ganz besonders bevorzugte organische Isocyanate sind.

Ferner entsteht im Rahmen der Ausführungsform mit Synthese von organischem Isocyanat neben besagtem organischen Isocyanat bevorzugt zusätzlich Chlorwasserstoff. Es ist wiederum bevorzugt diesen Chlorwasserstoff als Edukt der Herstellung von Chlor für die besagte Phosgensynthese zuzuführen. Die dafür notwendige Abtrennung und Reinigung des in der Isocyanat-Herstellung gebildeten Chlorwasserstoffs kann anschließend erfolgen durch eine oxidative Umsetzung in einer thermokatalytischen Gasphasenoxidation mit Sauerstoff zu Chlor und Wasser und ggf. Nutzung von O₂ aus der Wasserelektrolyse.

Eine weitere alternative Vorgehensweise bei der Nutzbarmachung des Chlorwasserstoffs als Edukt für die Herstellung von Chlor ist die Umsetzung des Chlorwasserstoffes mit Wasser zu Salzsäure und anschließender elektrochemischer Oxidation der Salzsäure zu Chlor und ggf. Wasserstoff. Diese wässrige Salzsäure wird insbesondere der zuvor beschriebenen elektrochemischen Oxidation nach der Salzsäure-Elektrolyse mit Gasdiffusionselektrode, der elektrochemischen Oxidation nach der Salzsäure-Diaphragmaelektrolyse oder der elektrochemischen Oxidation nach der Chloralkali-Elektrolyse) zugeführt. Im Falle der Salzsäure-Elektrolyse mit Gasdiffusionselektrode benötigte O₂ kann aus der Wasserelektrolyse bezogen werden.

Der anfallende Chlorwasserstoff aus der Isocyanat-Herstellung kann ebenso nach Reinigung und Absorption in Wasser zu Salzsäure umgesetzt und die resultierende Salzsäure in den Markt für diverse Anwendungen verkauft werden.

Im Rahmen einer besonders bevorzugten Ausführungsform des Verfahrens wird zusätzlich
eine Phosgensynthese durch Umsetzung von zumindest von Chlorgas und dem zuvor nach der Reinigung des besagten Kohlenmonoxid-haltigen Produktgases aus Schritt c) erhaltenem Kohlenmonoxid;
eine Isocyanat-Herstellung durch Umsetzung des erhaltenen Phosgens mit mindestens einem organischen Amin zu mindestens einer organischen Isocyanat-Verbindung und Chlorwasserstoff;
eine Abtrennung und Reinigung des in der Isocyanat-Herstellung gebildeten Chlorwasserstoffs und eine anschließende oxidative Umsetzung des Chlorwasserstoffs, ausgewählt aus
   - einer Umsetzung von Chlorwasserstoff in einer thermokatalytischen Gasphasenoxidation mit Sauerstoff zu Chlor und Wasser gegebenenfalls unter Nutzung von Sauerstoff aus der Wasserelektrolyse,
   - einer Umsetzung von Chlorwasserstoff mit Wasser zu Salzsäure und anschließender elektrochemischer Oxidation der Salzsäure zu Chlor und gegebenenfalls Wasserstoff;
eine an die oxidative Umsetzung des Chlorwasserstoffs anschließende Zuführung des gebildeten Chlors in die besagte Synthese von Phosgen, gegebenenfalls unter Zuführung einer zusätzlichen Menge Chlor in die besagte Synthese von Phosgen, die aus einer Chloralkali-Elektrolyse stammt;
durchgeführt.

Für alle Ausführungsformen des Verfahrens, die eine Phosgensynthese als Schritt enthalten, ist es weiter bevorzugt eine elektrochemische Oxidation (Elektrolyse) für die Chlorherstellung unter Verwendung von aus regenerativer Energie erzeugtem elektrischen Strom durchzuführen, insbesondere von elektrischem Strom wahlweise erhalten durch Nutzung von Windkraft, Sonnenenergie oder Wasserkraft.

Die in der Ausführungsform des Verfahrens für die Isocyanatherstellung eingesetzten organischen Amine werden wiederum bevorzugt durch einen zusätzlichen Schritt der Hydrierung von organischen Nitroverbindungen mit gasförmigem Wasserstoff bereitgestellt. Dabei eingesetzter Wasserstoff stammt bevorzugt aus einer Wasserelektrolyse und/oder aus der Reinigung des Kohlenmonoxid-haltigen Produktgases als abgetrennter Wasserstoff nach dem Schritt der Abtrennung von Kohlenmonoxid c4) und der Restgasbehandlung c5).

In einer besonders bevorzugten Ausführungsform des erfindungsgemäßen Verfahrens wird Wasserstoff aus einer Wasserelektrolyse in die Hydrierung von organischer Nitroverbindung für die Herstellung von organischem Amin eingespeist. Besonders bevorzugt wird dabei der Wasserstoff durch eine Wasserelektrolyse unter Verwendung von elektrischem Strom aus regenerativer Energie (insbesondere aus regenerativer Energie in Form von Windkraft, Sonnenenergie oder Wasserkraft) gebildet und einem Schritt zur Hydrierung von mindestens einer organischen Nitroverbindung zugeführt, wobei das bei der Hydrierung von besagter Nitroverbindung erhaltene mindestens eine organische Amin mit Phosgen unter Erhalt mindestens eines organischen Isocyanates umgesetzt wird.

Es ist erfindungsgemäß besonders bevorzugt, zumindest den bei der Reinigung aus dem Kohlenmonoxid-haltigen Produktgas abgetrennten Wasserstoff einem Hydrierungsschritt von mindestens einer organischen Nitroverbindung zuzuführen. Hierbei wird ebenso mindestens ein organisches Amin für die Herstellung von organischem Isocyanat zugänglich.

Die erhaltenen organischen Isocyanate können in einem zusätzlichen Schritt des Verfahrens mit mindestens einer organischen Verbindung mit mindestens zwei Hydroxygruppen, insbesondere mindestens einem Polyesterpolyol oder Polyetherpolyol, zu Polyurethan-Materialien umgesetzt werden. Hier gelten für die Herstellung diejenigen Ausführungsformen für die Bereitstellung von Polyurethan Material als bevorzugt, die zuvor zur Kennzeichnung der bevorzugt geeigneten polymeren, organischen Verbindung des in der partiellen Oxidation eingesetzten Materials beschrieben sind.

Entsprechend hergestellte Polyurethan-Materialien sind beispielsweise in Form von Schäumen, Lacken, Isoliermasse, Bestandteil von Marktprodukten. Sie können am Ende ihres Lebenszyklus als Material für die partielle Oxidation des erfindungsgemäßen Verfahrens dienen.

Ein weiterer Gegenstand der vorliegenden Erfindung ist eine Vorrichtung, mit der sich insbesondere das erfindungsgemäße Verfahren durchführen lässt. Dies ist eine Vorrichtung zur Bereitstellung von Kohlenmonoxid für die Herstellung von Phosgen, bevorzugt für die Herstellung von Phosgen zur Synthese organischer Isocyanatverbindung, enthaltend mindestens eine Dosiervorrichtung zur Einspeisung von Material, mindestens eine Vorrichtung zur partiellen Oxidation und mindestens eine Aufreinigungsvorrichtung für Kohlenmonoxid-haltiges Produktgas, dadurch gekennzeichnet, dass
i) besagte Vorrichtung zur partiellen Oxidation
   mindestens einen Auslass für einen Kohlenmonoxid-haltigen Produktgas-Strom
   und
   mindestens einen Reaktor für die partielle Oxidation des Materials mit mindestens einer Regelung enthält, die zur Temperatureinstellung des Reaktors auf eine Temperatur von mindestens 400°C geeignet ist, wobei der Reaktor mindestens einen Einlass für einen sauerstoffhaltigen Gasstrom und mindestens einen Einlass für Material und mindestens einen davon verschiedenen Auslass für Produktgas enthält, wobei der Einlass für einen sauerstoffhaltigen Gasstrom in Fluidverbindung mit einer Quelle eines sauerstoffhaltigen Gasstroms mit mindestens 50 Gew.-% Sauerstoffgas steht und der Auslass für Produktgas des Reaktor, gegebenenfalls zumindest über mindestens einen zwischengeschalteten Reaktor zur partiellen Oxidation des Produktgases, mit dem Auslass für den Kohlenmonoxid-haltigen Produktgas-Strom in Fluidverbindung steht; und,
ii) die Dosiervorrichtung und besagte Vorrichtung zur partiellen Oxidation derart zueinander angeordnet und ausgestaltet sind, dass die Dosiervorrichtung über mindestens eine Zuleitung mit dem Einlass für Material des Reaktors für die partielle Oxidation des Materials in Fluidverbindung steht; und
iii) eine Aufreinigungsvorrichtung für Kohlenmonoxid-haltiges Produktgas, welche mindestens einen Auslass für Kohlenmonoxid und mindestens einen Einlass für den Kohlenmonoxid-haltigen Produktgas-Strom enthält, wobei dieser Einlass in Fluidverbindung mit dem Auslass für den Kohlenmonoxid-haltigen Produktgas-Strom der Vorrichtung zur partiellen Oxidation steht, wobei die Aufreinigungsvorrichtung derart ausgestaltet ist, dass der Kohlenmonoxid-haltige Produktgasstrom zur Aufreinigung durch nachfolgend aufgeführte Module in der angegebenen Rangfolge hindurchgeführt werden kann
   iii-1) mindestens eine Gaswasch-Vorrichtung, enthaltend mindestens einen Einlass für den Kohlenmonoxid-haltigen Produktgas-Strom, mindestens einen Einlass für Wasser, mindestens einen Waschbehälter, in dem Wasser und Kohlenmonoxid-haltigen Produktgas-Strom zusammengeführt werden, sowie mindestens einen Auslass für Kohlenmonoxid-haltigen Produktgas-Strom und mindestens einen Auslass für Schlacke, wobei der Waschbehälter und der Auslass für Schlacke in Fluidverbindung stehen und der Waschbehälter und der Auslass für Kohlenmonoxid-haltigen Produktgas-Strom in Fluidverbindung stehen;
   iii-2) mindestens eine Trocken-Vorrichtung, enthaltend mindestens einen Einlass für Kohlenmonoxid-haltigen Produktgas-Strom, mindestens eine Kühlvorrichtung, die zur Abkühlung des Kohlenmonoxid-haltigen Produktgas-Stroms geeignet ist mindestens einen Auslass für Kohlenmonoxid-haltigen Produktgas-Strom sowie mindestens einen Auslass für flüssiges Kondensat,
   iii-3) mindestens eine Vorrichtung zur Abtrennung von Kohlendioxid, enthaltend mindestens einen Einlass für Kohlenmonoxid-haltigen Produktgas-Strom, mindestens einen Auslass für Kohlenmonoxid-haltigen Produktgas-Strom sowie zusätzlich mindestens einen Auslass für Kohlendioxid,
   iii-4) mindestens eine Trenneinheit zur Abtrennung von Kohlenmonoxid, enthaltend mindestens einen Einlass für Kohlenmonoxid-haltigen Produktgas-Strom, mindestens einen Auslass für Kohlenmonoxid sowie zusätzlich mindestens einen Auslass für Wasserstoffgas-haltiges Restgas.

Unter einer "Fluidverbindung" wird erfindungsgemäß ein Vorrichtungsteil verstanden, der Anlagenteile miteinander verbindet und durch die sich ein Stoff, der in jedem Aggregatzustand vorliegen kann, von einem Anlagenteil zu einem anderen Anlagenteil durch einen Stoffstrom transportieren lässt, beispielsweise eine Zuleitung in Form eines Rohres, die durch weitere Anlagenteile unterbrochen sein kann, oder eine Zuleitung in Form einer Fördervorrichtung, wie beispielsweise für Feststoffe eine Rutsche, eine Förderschnecke oder ein Fließband.

Bevorzugt werden die Reaktoren der Vorrichtung zur partiellen Oxidation ausgewählt aus mindestens einem Reaktor der Gruppe, die gebildet wird aus Reaktor für Flugstromvergasung, Reaktor für Wirbelschichtvergasung und Reaktor für Festbettvergasung.

Als Quelle eines sauerstoffhaltigen Gasstroms mit mindestens 50 Gew.-% Sauerstoffgas fungiert bevorzugt eine Vorrichtung zur Wasserelektrolyse, deren Auslass für Sauerstoffgas mit dem am Reaktor befindlichen Einlass für den sauerstoffhaltigen Gasstrom in Fluidverbindung steht. Es ist analog zum zuvor beschriebenen Verfahren im Rahmen einer bevorzugten Ausführungsform der Vorrichtung ebenfalls bevorzugt, wenn die erfindungsgemäße Vorrichtung zusätzlich eine Vorrichtung zur Wasserelektrolyse enthält, die neben mindestens einer Anode und mindestens einer Kathode, zusätzlich mindestens einen Einlass für Wasser, mindestens einen Auslass für Wasserstoffgas, mindestens einen Auslass für Sauerstoffgas aufweist und an eine Quelle für elektrischen Strom aus regenerativer Energie angeschlossen ist, und deren Auslass für Wasserstoffgas in Fluidverbindung mit einem Einlass für Wasserstoffgas einer Vorrichtung zur Hydrierung von organischer Nitroverbindung steht.

Die bevorzugten Ausführungsformen jeweils der Gaswasch-Vorrichtung, der Trocken-Vorrichtung, der Vorrichtung zur Abtrennung von Kohlendioxid und der Trenneinheit zur Abtrennung von Kohlenmonoxid werden derart ausgestaltet, wie unter dem Gegenstand des erfindungsgemäßen Verfahrens beschrieben (vide supra).

Es hat sich als bevorzugt erwiesen, wenn im Rahmen einer bevorzugten Ausführungsform die Vorrichtung sich dadurch gekennzeichnet, dass die Aufreinigungsvorrichtung zusätzlich mindestens eine Vorrichtung zur Restgasbehandlung enthält, enthaltend mindestens einen Einlass für Wasserstoff-haltiges Restgas, mindestens einen Auslass für Wasserstoffgas und mindestens einen Auslass für Endgas, wobei der Einlass für Wasserstoff-haltiges Restgas in Fluidverbindung mit dem Auslass für Wasserstoff-haltiges Restgas der Trenneinheit zur Abtrennung von Kohlenmonoxid steht.

Eine ganz besonders bevorzugte Ausführungsform der Vorrichtung eignet sich zur Rückführung von in der Vorrichtung zur Abtrennung von Kohlendioxid abgetrenntem CO₂ in einen Reaktor der partiellen Oxidation. Daher ist eine ganz bevorzugte Ausführungsform der Vorrichtung dadurch gekennzeichnet, dass mindestens ein Reaktor der Vorrichtung zur partiellen Oxidation einen Einlass für Kohlendioxid aufweist, der in einer Fluidverbindung mit dem Auslass für Kohlendioxid der Vorrichtung zur Abtrennung von Kohlendioxid steht, wodurch eine Rückführung des Kohlendioxids in diesen Reaktor der Vorrichtung zur partiellen Oxidation entgegen der Fließrichtung des Kohlenmonoxid-haltigen Produktgasstroms ermöglicht wird.

Die Verfahrensführung einer mindestens zweistufigen partiellen Oxidation hat sich als bevorzugt erwiesen. Dafür eignet sich eine Ausführungsform der Vorrichtung, in der besagte Vorrichtung zur partiellen Oxidation
mindestens einen Auslass für einen Kohlenmonoxid-haltigen Produktgas-Strom
und
einen ersten Reaktor für die partielle Oxidation des Materials mit mindestens einem Heizelement, das zur Temperierung des Reaktors auf eine Temperatur von mindestens 400°C geeignet ist, wobei der erste Reaktor mindestens einen Einlass für einen sauerstoffhaltigen Gasstrom und mindestens einen Einlass für Material und mindestens einen davon verschiedenen Auslass für Produktgas enthält, wobei der Einlass für einen sauerstoffhaltigen Gasstrom in Fluidverbindung mit einer Quelle eines sauerstoffhaltigen Gasstroms mit mindestens 50 Gew.-% Sauerstoffgas steht;
und
einen zweiten Reaktor für die partielle Oxidation des Produktgases mit mindestens einem Heizelement, das zur Temperierung des Reaktors auf eine Temperatur von mehr als 800°C geeignet ist, enthält, wobei der zweite Reaktor mindestens einen Einlass für einen sauerstoffhaltigen Gasstrom und mindestens einen Einlass für gegebenenfalls zwischenbehandeltes Produktgas des ersten Reaktors und mindestens einen davon verschiedenen Auslass für Produktgas enthält, wobei der der Auslass für Produktgas des ersten Reaktors mit dem Einlass des zweiten Reaktors für gegebenenfalls zwischenbehandeltes Produktgas des ersten Reaktors in Fluidverbindung steht, und der Einlass für einen sauerstoffhaltigen Gasstrom in Fluidverbindung mit einer Quelle eines sauerstoffhaltigen Gasstroms mit mindestens 50 Gew.-% Sauerstoffgas steht, und der Auslass für Produktgas des zweiten Reaktors mit dem Auslass für den Kohlenmonoxid-haltigen Produktgas-Strom in Fluidverbindung steht.

Bevorzugt ist ein Reaktor derart ausgestaltet, dass neben dem sauerstoffhaltigem Gasstrom zusätzlich gasförmiges Wasser in den Reaktor eingebracht werden kann. Solche Reaktoren sind dadurch gekennzeichnet, dass mindestens ein Reaktor in Fluidverbindung mit mindestens einer Quelle für gasförmiges Wasser steht.

Weiterhin ist eine Ausführungsform der Vorrichtung bevorzugt, in der der Auslass für Kohlenmonoxid der Trenneinheit für Kohlenmonoxid in Fluidverbindung mit dem Einlass eines Reaktors einer Vorrichtung zur Phosgensynthese steht. Die Vorrichtung zur Phosgensynthese enthält mindestens einen Einlass für Chlorgas, mindestens einen Einlass für Kohlenmonoxid und mindestens einen Auslass für Phosgen.

Für die Synthese von organischem Isocyanat ist es im Rahmen einer bevorzugten Ausführungsform der Vorrichtung vorteilhaft, wenn der Auslass für Phosgen der Vorrichtung zur Phosgensynthese mit einer Vorrichtung zur Herstellung von organischem Isocyanat in Fluidverbindung steht, wobei die Vorrichtung zur Herstellung von organischem Isocyanat neben mindestens einem Einlass für Phosgen zusätzlich mindestens einen Einlass für organisches Amin und mindestens einen Auslass für organisches Isocyanat besitzt. Dabei ist es besonders bevorzugt, wenn die Vorrichtung zur Herstellung von organischem Isocyanat über mindestens einen Einlass für organisches Amin mit einer Vorrichtung zur Hydrierung von organischer Nitroverbindung in Fluidverbindung steht, wobei die Vorrichtung zur Hydrierung organischer Nitroverbindung mindestens einen Einlass für Wasserstoffgas, mindestens einen Einlass für organische Nitroverbindung und mindestens einen Auslass für organisches Amin aufweist. Als Quelle für das Wasserstoffgas zur Hydrierung kann vorteilhafter Weise das Wasserstoffgas aus der Restgasbehandlung des Restgases der Vorrichtung der H₂-CO Trennung dienen. Zu diesem Zweck stehen bevorzugt der Auslass für Wasserstoffgas der Vorrichtung zur Restgasbehandlung und der Einlass für Wasserstoffgas der Vorrichtung zur Hydrierung von organischer Nitroverbindung in Fluidverbindung. Zusätzlich oder in Ergänzung dazu dient besonders bevorzugt als Quelle für das Wasserstoffgas zur Hydrierung das Wasserstoffgas aus der Vorrichtung zur Herstellung von Chlor. Zu diesem Zweck stehen besonders bevorzugt der Auslass für Wasserstoffgas der Vorrichtung zur Herstellung von Chlor und der Einlass für Wasserstoffgas der Vorrichtung zur Hydrierung von organischer Nitroverbindung in Fluidverbindung.

Für eine Ausnutzung des Wasserstoffgases aus dem Restgas der Vorrichtung der H₂-CO Trennung für Synthesezwecke ist es bevorzugt, wenn der Auslass für das Restgas der Vorrichtung der H₂-CO Trennung mit dem Einlass einer Vorrichtung zur Restgasbehandlung in Fluidverbindung steht, wobei besagte Vorrichtung zur Restgasbehandlung mindestens einen Auslass für Wasserstoffgas und mindestens einen Auslass für Restgas der Restgasbehandlung besitzt. In diesem Falle ist es bevorzugt, wenn der Auslass für das Restgas der Vorrichtung für die Restgasbehandlung in Fluidverbindung mit dem Heizelement des Reformers, insbesondere mit der Brennkammer des Heizelementes, steht.

Ein weiterer Gegenstand der Erfindung ist die Verwendung einer partiellen Oxidation von Material, enthaltend mindestens eine polymere, organische Verbindung, zur Bereitgestellung von Kohlenmonoxid für die Synthese von Phosgen. Dabei ist es bevorzugt, wenn die partielle Oxidation des Materials gemäß eines Verfahrens des ersten Erfindungsgegenstandes erfolgt.

Ein weiterer Gegenstand der Erfindung ist die Verwendung von Material, enthaltend mindestens eine polymere, organische Verbindung, in einer partiellen Oxidation des Materials zur Bereitgestellung von Kohlenmonoxid für die Synthese von Phosgen. Dabei ist es bevorzugt, wenn die partielle Oxidation des Materials gemäß eines Verfahrens des ersten Erfindungsgegenstandes erfolgt.

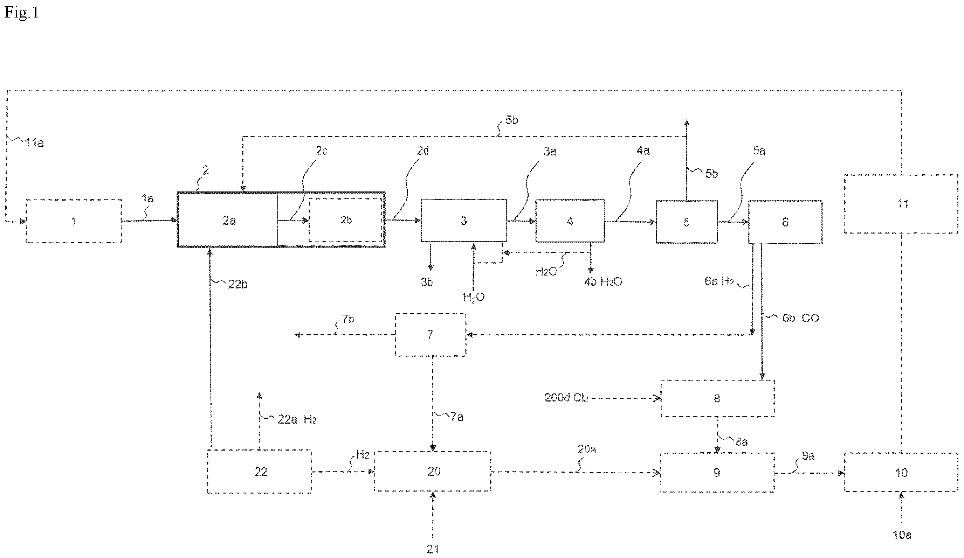

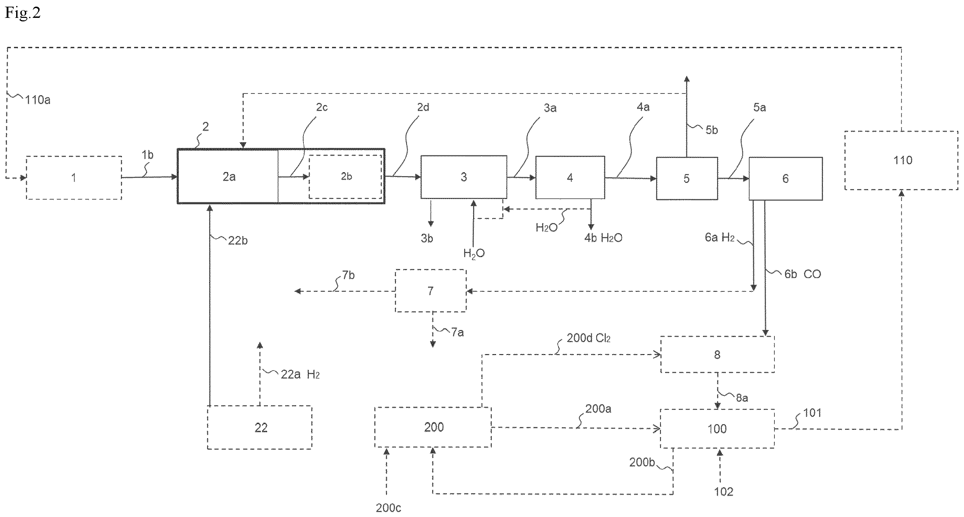

Die Erfindung wird nachstehend anhand der Figuren 1 und 2 beispielhaft näher erläutert. In den Figuren haben die folgenden Bezugszeichen die jeweils rechtsstehende Bedeutung:
- 1: Material Vorbehandlung
- 1a: aufbereitetes Material, enthaltend Polyurethan als eine polymere, organische Verbindung
- 1b: aufbereitetes Material, enthaltend Polycarbonat als eine polymere, organische Verbindung
- 2: partielle Oxidation (Gasifikation)
- 2a: Reaktor zur partiellen Oxidation
- 2b: Reaktor zur partiellen Oxidation des Produktgases aus 2a
- 2c: Produktgas
- 2d: Produktgasstrom, der CO- und H2-haltig ist und partikelförmigen Feststoff enthält
- 3: Waschschritt
- 3a: Produktgasstrom, der CO- und H2-haltig und gewaschen ist
- 3b: Schlacke
- 4: Trocknung durch H₂O Entfernung
- 4a: Produktgasstrom, der CO- und H2-haltig, sowie getrocknet ist
- 4b: Wasser
- 5: Abtrennung von Kohlendioxid
- 5a: Produktgasstrom aus der CO₂-Entfernung, der CO- und H2-haltig ist
- 5b: CO₂
- 6: Abtrennung von Kohlenmonoxid (H₂-CO Trennung)
- 6a: Restgas
- 6b: CO
- 7: H₂ Reinigung
- 7a: H₂
- 7b: Endgas
- 8: Phosgensynthese
- 8a: Phosgen
- 9: Isocyanat-Herstellung
- 9a: organisches Isocyanat
- 10: Polyurethan-Herstellung
- 10a: organische Verbindung mit mindestens 2 Hydroxygruppen, z.B. Polyetherpolyol oder Polyesterpolyol
- 11: Polyurethan end of life - Sammlung / Trennung
- 11a: Polyurethan haltiges Material
- 20: Hydrierung organischer Nitroverbindungen
- 20a: organische Diaminoverbindung
- 20b: organische Verbindung
- 21: organische Nitroverbindung
- 22: Wasserelektrolyse
- 22a: Wasserstoff
- 22b: Sauerstoff
- 30: H₂O für die partielle Oxidation (Gasifikation) 2
- 100: Polycarbonat-Herstellung
- 101: Polycarbonat
- 110: Polycarbonat-haltiges Material end of life - Sammlung / Trennung
- 110a: Polycarbonat haltiges Material
- 200: Chloralkali-Elektrolyse
- 200a: Natronlauge
- 200b: Natriumchlorid-haltiges Abwasser aus 100
- 200c: Natriumchlorid (NaCl)
- 200d: Chlorgas

Pfeile in den Figuren symbolisieren den Fluss von Stoffen, Energie oder Wärme zwischen Verfahrensschritten bzw. Vorrichtungsteilen, in denen die entsprechenden Verfahrensschritte ablaufen. Gestrichelte Pfeile symbolisieren zusätzliche Merkmale bevorzugter Ausführungsformen des Verfahrens, die bevorzugt einzeln oder in Kombination eingesetzt werden können.

Fig.1 zeigt eine Ausführungsform des Verfahrens für die Herstellung von Kohlenmonoxid durch Gasifikation einer Polyurethan-Abfallfraktion unter Nutzung von Sauerstoff aus einer Wasserelektrolyse für die Darstellung von Phosgen zur Synthese von organischem Isocyanat, enthaltend zumindest die Schritte
a) Bereitstellung eines sauerstoffhaltigen Gasstroms **22b**, enthaltend mindestens 50 Gew.-% Sauerstoffgas,
b) partielle Oxidation **2** von Material **1a**, enthaltend mindestens eine polymere, organische Verbindung, wobei besagtes Material **1a** in einen Reaktor **2a** einer Vorrichtung eingebracht und dort zumindest durch Zufuhr des besagten sauerstoffhaltigen Gasstromes **22b** und von Wärme bei mindestens 400°C unter Bildung eines Produktgases **2c** behandelt und das resultierende Produktgas **2c**, gegebenenfalls nach mindestens einem weiteren partiellen Oxidationsschritt **2b** des Produktgases **2c**, als ein Kohlenmonoxid-haltiger Produktgas-Strom **2d** gemeinsam mit darin dispergiertem, partikelförmigem Feststoff aus der Vorrichtung ausgebracht wird;
c) der Kohlenmonoxid-haltige Produktgas-Strom **2d** einer Reinigung zugeführt wird, in der zumindest
   c1) der Kohlenmonoxid-haltige Produktgas-Strom **2d** zur Abtrennung von Feststoff einem Waschschritt **3** zugeführt wird, worin
      (i) Wasser mit dem Kohlenmonoxid-haltigen Produktgas-Strom **2d** in Kontakt gebracht wird, wodurch der im Kohlenmonoxid-haltigen Produktgas-Strom **2d** dispergierte, partikelförmige Feststoff eine Schlacke **3b** bildet und diese Schlacke **3b** entfernt und abgeführt wird,
      (ii) der von partikelförmigem Feststoff gereinigte, Kohlenmonoxid-haltige Produktgas-Strom **3a** ausgebracht wird;
   c2) ein mittels Waschschritt **3** von partikelförmigem Feststoff gereinigter Kohlenmonoxid-haltiger Produktgas-Strom **3a** in einem Trockenschritt zumindest einer Abtrennung von Wasser **4** zugeführt, Wasser **4b** abgetrennt und der resultierende Kohlenmonoxid-haltige Produktgas-Strom **4a** ausgebracht wird,
   c3) ein mittels Abtrennung von Wasser **4** gereinigter Kohlenmonoxid-haltiger Produktgas-Strom **4a** zumindest einer Abtrennung von Kohlendioxid **5** zugeführt, Kohlendioxid abgetrennt und der resultierende Kohlenmonoxid-haltige Produktgas-Strom **5a** und Kohlendioxid **5b** ausgebracht wird, wobei das Kohlendioxid **5b** zumindest teilweise in einen Reaktor der partiellen Oxidation **2** eingebracht wird;
   c4) ein mittels Abtrennung von Kohlendioxid gereinigter Kohlenmonoxid-haltiger Produktgas-Strom **5a** zumindest einer Trenneinheit **6** zur Abtrennung von Kohlenmonoxid zugeführt, eine Abtrennung von Kohlenmonoxid durchgeführt und das resultierende Kohlenmonoxid **6b** und ein Wasserstoffgas-haltiges Restgas **6a** ausgebracht wird;
   c5) optional ein mittels besagter Trenneinheit **6** abgetrenntes, Wasserstoffgas-haltiges Restgas **6a** einer Restgasbehandlung **7** zugeführt, Wasserstoffgas **7a** abgetrennt und Wasserstoffgas **7a** und Endgas **7b** ausgebracht wird;
d) Phosgensynthese **8** durch Umsetzung von zumindest Chlorgas und Kohlenmonoxid, wobei als Kohlenmonoxid mindestens das Kohlenmonoxid **6b** aus der Reinigung des Schrittes c) umgesetzt und Phosgen **8a** ausgebracht wird.

Die Einbindung dieses Verfahrens in eine Prouktion von Isocyanat wird exemplarisch in der Beispielsektion beschrieben.

Fig.2 zeigt eine Ausführungsform des Verfahrens für die Herstellung von Kohlenmonoxid durch Gasifikation einer Polycarbonat-Abfallfraktion unter Nutzung von Sauerstoff aus einer Wasserelektrolyse für die Darstellung von Phosgen zur Synthese von Polycarbonat. Welches zumindest die unter Fig.1 beschriebenen Schritte außer Schritt d) enthält.

Die Einbindung dieses Verfahrens in eine Produktion von Polycarbonat wird exemplarisch in der Beispielsektion beschrieben.

Die nachfolgenden Aspekte 1 bis 25 stellen eine weitere Ausführungsform der Erfindung dar, wobei die Bezugszeichen in Klammern lediglich klarstellenden Charakter haben und die Ausführungsform nicht auf den Gegenstand der Figur 1 einschränkt:
1. Verfahren zur Bereitstellung von Kohlenmonoxid für die Herstellung von Phosgen, bevorzugt für die Herstellung von Phosgen zur Synthese organischer Isocyanatverbindung, enthaltend mindestens folgende Schritte
   a) Bereitstellung eines sauerstoffhaltigen Gasstroms (22b), enthaltend mindestens 50 Gew.-% Sauerstoffgas,
   b) partielle Oxidation (2) von Material (1a), enthaltend mindestens eine polymere, organische Verbindung, wobei besagtes Material (1a) in einen Reaktor (2a) einer Vorrichtung eingebracht und dort zumindest durch Zufuhr des besagten sauerstoffhaltigen Gasstromes (22b) und von Wärme bei mindestens 400°C unter Bildung eines Produktgases (2c) behandelt und das resultierende Produktgas (2c), gegebenenfalls nach mindestens einem weiteren partiellen Oxidationsschritt (2b) des Produktgases (2c), als ein Kohlenmonoxid-haltiger Produktgas-Strom (2d) gemeinsam mit darin dispergiertem, partikelförmigem Feststoff aus der Vorrichtung ausgebracht wird;
   c) der Kohlenmonoxid-haltige Produktgas-Strom (2d) einer Reinigung zugeführt wird, in der zumindest
      c1) der Kohlenmonoxid-haltige Produktgas-Strom (2d) zur Abtrennung von Feststoff einem Waschschritt (3) zugeführt wird, worin
         (i) Wasser mit dem Kohlenmonoxid-haltigen Produktgas-Strom (2d) in Kontakt gebracht wird, wodurch der im Kohlenmonoxid-haltigen Produktgas-Strom (2d) dispergierte, partikelförmige Feststoff eine Schlacke (3b) bildet und diese Schlacke (3b) entfernt und abgeführt wird,
         (ii) der von partikelförmigem Feststoff gereinigte, Kohlenmonoxid-haltige Produktgas-Strom (3a) ausgebracht wird;
      c2) ein mittels Waschschritt (3) von partikelförmigem Feststoff gereinigter Kohlenmonoxid-haltiger Produktgas-Strom (3a) in einem Trockenschritt zumindest einer Abtrennung von Wasser (4) zugeführt, Wasser (4b) abgetrennt und der resultierende Kohlenmonoxid-haltige Produktgas-Strom (4a) ausgebracht wird,
      c3) ein mittels Abtrennung von Wasser (4) gereinigter Kohlenmonoxid-haltiger Produktgas-Strom (4a) zumindest einer Abtrennung von Kohlendioxid (5) zugeführt, Kohlendioxid abgetrennt und der resultierende Kohlenmonoxid-haltige Produktgas-Strom (5a) und Kohlendioxid (5b) ausgebracht wird;
      c4) ein mittels Abtrennung von Kohlendioxid gereinigter Kohlenmonoxid-haltiger Produktgas-Strom (5a) zumindest einer Trenneinheit (6) zur Abtrennung von Kohlenmonoxid zugeführt, eine Abtrennung von Kohlenmonoxid durchgeführt und das resultierende Kohlenmonoxid (6b) und ein Wasserstoffgas-haltiges Restgas (6a) ausgebracht wird;
      c5) optional ein mittels besagter Trenneinheit (6) abgetrenntes, Wasserstoffgas-haltiges Restgas (6a) einer Restgasbehandlung (7) zugeführt, Wasserstoffgas (7a) abgetrennt und Wasserstoffgas (7a) und Endgas (7b) ausgebracht wird;
   d) gegebenenfalls Phosgensynthese (8) durch Umsetzung von zumindest Chlorgas und Kohlenmonoxid, wobei als Kohlenmonoxid mindestens das Kohlenmonoxid (6b) aus der Reinigung des Schrittes c) umgesetzt und Phosgen (8a) ausgebracht wird.
2. Verfahren nach Aspekt 1, dadurch gekennzeichnet, dass zur Bereitstellung des sauerstoffhaltigen Gasstroms (22b) eine Elektrolyse von Wasser (22) unter Erhalt von Sauerstoffgas (22a) und Wasserstoffgas durchgeführt wird und das Sauerstoffgas (22a) aus dieser Elektrolyse (22) zur Bereitstellung des sauerstoffhaltigen Gasstromes (22b) genutzt wird.
3. Verfahren nach einem der Aspekte 1 oder 2, dadurch gekennzeichnet, dass dem Reaktor (2a) zusätzlich gasförmiges Wasser zugeführt wird, in einem Gewichtsverhältnis von gasförmigem Wasser zu Sauerstoffgas des sauerstoffhaltigen Gasstromes von mindestens 0,2.
4. Verfahren nach einem der vorangehenden Aspekte, dadurch gekennzeichnet, dass die partielle Oxidation (2) bei einem absoluten Druck von mehr als 1 bar, bevorzugt bei einem absoluten Druck in einem Bereich von 2 bis 80 bar, besonders bevorzugt bei einem absoluten Druck in einem Bereich von 2 bis 50 bar, durchgeführt wird.
3. Verfahren nach einem der vorangehenden Aspekte, dadurch gekennzeichnet, dass die Gesamtmenge aller in dem Material (1a) enthaltenden polymeren, organischen Verbindungen mindestens 50 Gew.-%, bevorzugt von mindestens 60 Gew.-%, besonders bevorzugt von mindestens 75 Gew.-%, beträgt.
4. Verfahren nach einem der vorangehenden Aspekte, dadurch gekennzeichnet, dass die Gesamtmenge aller in dem Material (1a) enthaltenden polymeren, organischen Verbindungen einen Kohlenstoffanteil von mindestens 40,0 Gew.%, bevorzugt von mindestens 50 Gew.-%, aufweist.
5. Verfahren nach einem der vorangehenden Aspekte, dadurch gekennzeichnet, dass das Gewichtsverhältnis des im sauerstoffhaltigen Gasstrom (22b) enthaltenen Sauerstoffgases zur polymeren, organischen Verbindung innerhalb eines Gewichtsverhältnisbereiches von 0,4 zu 1,0 bis 1,2 zu 1,0, bevorzugt von 0,6 zu 1,0 bis 0,9 zu 1,0 in den Reaktor (2a) eingebracht werden.
6. Verfahren nach einem der vorangehenden Aspekte, dadurch gekennzeichnet, dass die polymere, organische Verbindung des Materials (1a) ausgewählt wird aus mindestens einer homopolymeren oder copolymeren Verbindung aus der Gruppe Cellulose, Polyester, Polyamid, Polyurethan (PUR), Polyharnstoff, Polyisocyanurat (PIR), Polycarbonat, bevorzugt aus mindestens einer homopolymeren oder copolymeren Verbindung der Gruppe Polyurethan (PUR), Polyisocyanurat (PIR).
7. Verfahren nach einem der vorangehenden Aspekte, dadurch gekennzeichnet, dass die Struktur der polymeren, organischen Verbindung des Materials (1a) mindestens eine sich wiederholende Struktureinheit der Formel (I) enthält, 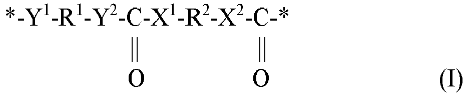 worin
   X¹ und X² unabhängig voneinander eine direkte Bindung, O oder NH, bevorzugt O oder NH, bedeuten,
   Y¹ und Y² unabhängig voneinander für O oder NH stehen,
   R¹ und R² unabhängig voneinander jeweils für ein beliebiges bivalentes, Kohlenstoff enthaltendes Strukturelement stehen, und
   eine mit ^{∗} gekennzeichnete kovalente Bindung eine Bindung zum Restmolekül der polymeren, organischen Verbindung bedeutet.
8. Verfahren nach Aspekt 7, dadurch gekennzeichnet, dass die Gesamtmenge der in dem Material (1a) enthaltenden polymeren, organischen Verbindungen mit mindestens einer sich wiederholenden Struktureinheit der Formel (I) mindestens 50 Gew.-%, bevorzugt von mindestens 60 Gew.-%, besonders bevorzugt von mindestens 75 Gew.-%, beträgt.
9. Verfahren nach einem der vorangehenden Aspekte, dadurch gekennzeichnet, dass in die partielle Oxidation des Materials (1a) in dem Reaktor (2a) bei einer Temperatur in einem Temperaturbereich von 600 °C bis 1500°C, insbesondere von 850°C bis 1400°C, weiter bevorzugt von 1100°C bis 1300°C, durchgeführt wird.
10. Verfahren nach einem der vorangehenden Aspekte, dadurch gekennzeichnet, dass das Wasser beim Waschschritt (3) in den Kohlenmonoxid-haltigen Produktgas-Strom (2c) gesprüht wird.
11. Verfahren nach einem der vorangehenden Aspekte, dadurch gekennzeichnet, dass der aus dem Waschschritt (3) ausgebrachte, von partikelförmigem Feststoff gereinigte, Kohlenmonoxid-haltige Produktgas-Strom (3a) eine Temperatur von höchstens 100 °C bevorzugt höchstens 80°C, hat.
12. Verfahren nach einem der vorangehenden Aspekte, dadurch gekennzeichnet, dass die partielle Oxidation (2) des Materials (1a) in mindestens zwei Schritten durchgeführt wird, wobei in einem ersten Schritt eine partielle Oxidation des Materials (1a) in einem ersten Reaktor (2a) unter Zuführung eines sauerstoffhaltigen Gasstromes (22b) bei einer Temperatur in einem Temperaturbereich von 400 °C bis 800°C durchgeführt wird, das Produktgas (2c) des ersten Reaktors in einen zweiten Reaktor (2b) überführt wird und dort in einem weiteren Schritt unter Zuführung eines sauerstoffhaltigen Gasstromes (22b) einer partiellen Oxidation bei einer Temperatur von mehr als 800 °C, bevorzugt bei einer Temperatur in einem Bereich von 1300°C bis 1500°C, unterworfen wird unter Erhalt des Kohlenmonoxid-haltigen Produktgas-Stroms (2d).
13. Verfahren nach einem der Aspekte 1 bis 12, dadurch gekennzeichnet, dass das in Schritt c3) ausgebrachte, abgetrennte Kohlendioxid (5b) zu einem Reaktor der partiellen Oxidation (2) zurückgeführt und in diesen Reaktor eingebracht wird.
14. Verfahren nach Aspekt 13, dadurch gekennzeichnet, dass die partielle Oxidation (2) des Materials (1a) gemäß Anspruch 12 durchgeführt wird und dass das in Schritt c3) ausgebrachte, abgetrennte Kohlendioxid (5b) zumindest zum ersten Reaktor (2a) der partiellen Oxidation zurückgeführt und in diesen Reaktor eingebracht wird.
15. Verfahren nach einem der vorangehenden Aspekte, dadurch gekennzeichnet, dass der partiellen Oxidation (2) das Kohlendioxid (5b) in einer Menge von höchstens 1 kg pro kg polymerer, organischer Verbindung, bevorzugt von höchstens 0,9 kg pro kg polymerer, organischer Verbindung, besonders bevorzugt von 0,8 kg pro kg polymerer, organischer Verbindung, zugefügt wird.
16. Verfahren nach einem der vorangehenden Aspekte, dadurch gekennzeichnet, dass die Phosgensynthese (8) in Schritt d) und zusätzlich folgender Schritt ausgeführt wird:
   e) Herstellung von organischem Isocyanat (9), worin das Phosgen (8a) aus der Phosgensynthese (8) mit mindestens einer organischen Aminoverbindung (20a) umgesetzt und zumindest organisches Isocyanat (9a) ausgebracht wird.
17. Vorrichtung zur Bereitstellung von Kohlenmonoxid für die Herstellung von Phosgen, bevorzugt für die Herstellung von Phosgen zur Synthese organischer Isocyanatverbindung, enthaltend mindestens eine Dosiervorrichtung zur Einspeisung von Material, mindestens eine Vorrichtung zur partiellen Oxidation und mindestens eine Aufreinigungsvorrichtung für Kohlenmonoxid-haltiges Produktgas, dadurch gekennzeichnet, dass
   i) besagte Vorrichtung zur partiellen Oxidation
      mindestens einen Auslass für einen Kohlenmonoxid-haltigen Produktgas-Strom
      und
      mindestens einen Reaktor für die partielle Oxidation des Materials mit mindestens einem Heizelement enthält, das zur Temperierung des Reaktors auf eine Temperatur von mindestens 400°C geeignet ist, wobei der Reaktor mindestens einen Einlass für einen sauerstoffhaltigen Gasstrom und mindestens einen Einlass für Material und mindestens einen davon verschiedenen Auslass für Produktgas enthält, wobei der Einlass für einen sauerstoffhaltigen Gasstrom in Fluidverbindung mit einer Quelle eines sauerstoffhaltigen Gasstroms mit mindestens 50 Gew.-% Sauerstoffgas steht und der Auslass für Produktgas des Reaktors, gegebenenfalls zumindest über mindestens einen zwischengeschalteten Reaktor zur partiellen Oxidation des Produktgases, mit dem Auslass für den Kohlenmonoxid-haltigen Produktgas-Strom in Fluidverbindung steht;
      und,
   ii) die Dosiervorrichtung und besagte Vorrichtung zur partiellen Oxidation derart zueinander angeordnet und ausgestaltet sind, dass die Dosiervorrichtung über mindestens eine Zuleitung mit dem Einlass für Material des Reaktors für die partielle Oxidation des Materials in Fluidverbindung steht; und
   iii) eine Aufreinigungsvorrichtung für Kohlenmonoxid-haltiges Produktgas, welche mindestens einen Auslass für Kohlenmonoxid und mindestens einen Einlass für den Kohlenmonoxid-haltigen Produktgas-Strom enthält, wobei dieser Einlass in Fluidverbindung mit dem Auslass für den Kohlenmonoxid-haltigen Produktgas-Strom der Vorrichtung zur partiellen Oxidation steht, wobei die Aufreinigungsvorrichtung derart ausgestaltet ist, dass der Kohlenmonoxid-haltige Produktgasstrom zur Aufreinigung durch nachfolgend aufgeführte Module in der angegebenen Rangfolge hindurchgeführt werden kann
      iii-1) mindestens eine Gaswasch-Vorrichtung, enthaltend mindestens einen Einlass für den Kohlenmonoxid-haltigen Produktgas-Strom, mindestens einen Einlass für Wasser, mindestens einen Waschbehälter, in dem Wasser und Kohlenmonoxid-haltigen Produktgas-Strom zusammengeführt werden, sowie mindestens einen Auslass für Kohlenmonoxid-haltigen Produktgas-Strom und mindestens einen Auslass für Schlacke, wobei der Waschbehälter und der Auslass für Schlacke in Fluidverbindung stehen und der Waschbehälter und der Auslass für Kohlenmonoxid-haltigen Produktgas-Strom in Fluidverbindung stehen;
      iii-2) mindestens eine Trocken-Vorrichtung, enthaltend mindestens einen Einlass für Kohlenmonoxid-haltigen Produktgas-Strom, mindestens eine Kühlvorrichtung, die zur Abkühlung des Kohlenmonoxid-haltigen Produktgas-Stroms geeignet ist mindestens einen Auslass für Kohlenmonoxid-haltigen Produktgas-Strom sowie mindestens einen Auslass für flüssiges Kondensat,
      iii-3) mindestens eine Vorrichtung zur Abtrennung von Kohlendioxid, enthaltend mindestens einen Einlass für Kohlenmonoxid-haltigen Produktgas-Strom, mindestens einen Auslass für Kohlenmonoxid-haltigen Produktgas-Strom sowie zusätzlich mindestens einen Auslass für Kohlendioxid,
      iii-4) mindestens eine Trenneinheit zur Abtrennung von Kohlenmonoxid, enthaltend mindestens einen Einlass für Kohlenmonoxid-haltigen Produktgas-Strom, mindestens einen Auslass für Kohlenmonoxid sowie zusätzlich mindestens einen Auslass für Wasserstoffgas-haltiges Restgas.
18. Vorrichtung nach Aspekt 17, dadurch gekennzeichnet, dass die Aufreinigungsvorrichtung zusätzlich mindestens eine Vorrichtung zur Restgasbehandlung enthält, enthaltend mindestens einen Einlass für Wasserstoff-haltiges Restgas, mindestens einen Auslass für Wasserstoffgas und mindestens einen Auslass für Endgas, wobei der Einlass für Wasserstoff-haltiges Restgas in Fluidverbindung mit dem Auslass für Wasserstoff-haltiges Restgas der Trenneinheit zur Abtrennung von Kohlenmonoxid steht.
19. Vorrichtung nach einem der Aspekte 17 oder 18, dadurch gekennzeichnet, dass mindestens ein Reaktor der Vorrichtung zur partiellen Oxidation einen Einlass für Kohlendioxid aufweist, der in einer Fluidverbindung mit dem Auslass für Kohlendioxid der Vorrichtung zur Abtrennung von Kohlendioxid steht, wodurch eine Rückführung des Kohlendioxids in diesen Reaktor der Vorrichtung zur partiellen Oxidation entgegen der Fließrichtung des Kohlenmonoxid-haltigen Produktgasstroms ermöglicht wird.
20. Vorrichtung nach einem der Aspekte 17 bis 19, dadurch gekennzeichnet, dass besagte Vorrichtung zur partiellen Oxidation
   mindestens einen Auslass für einen Kohlenmonoxid-haltigen Produktgas-Strom
   und
   einen ersten Reaktor für die partielle Oxidation des Materials mit mindestens einer Regelung enthält, die zur Temperatureinstellung des Reaktors auf eine Temperatur von mindestens 400°C geeignet ist, wobei der erste Reaktor mindestens einen Einlass für einen sauerstoffhaltigen Gasstrom und mindestens einen Einlass für Material und mindestens einen davon verschiedenen Auslass für Produktgas enthält, wobei der Einlass für einen sauerstoffhaltigen Gasstrom in Fluidverbindung mit einer Quelle eines sauerstoffhaltigen Gasstroms mit mindestens 50 Gew.-% Sauerstoffgas steht;
   und
   einen zweiten Reaktor für die partielle Oxidation des Produktgases mit mindestens einem Heizelement, das zur Temperierung des Reaktors auf eine Temperatur von mehr als 800°C geeignet ist, enthält, wobei der zweite Reaktor mindestens einen Einlass für einen sauerstoffhaltigen Gasstrom und mindestens einen Einlass für gegebenenfalls zwischenbehandeltes Produktgas des ersten Reaktors und mindestens einen davon verschiedenen Auslass für Produktgas enthält, wobei der der Auslass für Produktgas des ersten Reaktors mit dem Einlass des zweiten Reaktors für gegebenenfalls zwischenbehandeltes Produktgas des ersten Reaktors in Fluidverbindung steht, und der Einlass für einen sauerstoffhaltigen Gasstrom in Fluidverbindung mit einer Quelle eines sauerstoffhaltigen Gasstroms mit mindestens 50 Gew.-% Sauerstoffgas steht, und der Auslass für Produktgas des zweiten Reaktors mit dem Auslass für den Kohlenmonoxid-haltigen Produktgas-Strom in Fluidverbindung steht.
21. Vorrichtung nach einem der Aspekte 17 bis 20, dadurch gekennzeichnet, dass mindestens ein Reaktor in Fluidverbindung mit mindestens einer Quelle für gasförmiges Wasser steht.
22. Vorrichtung nach einem der Aspekte 17 bis 21, dadurch gekennzeichnet, dass der Auslass für Kohlenmonoxid der Trenneinheit für Kohlenmonoxid in Fluidverbindung mit dem Einlass eines Reaktors einer Vorrichtung zur Phosgensynthese steht.
23. Verwendung einer partiellen Oxidation von Material (2), enthaltend mindestens eine polymere, organische Verbindung, zur Bereitgestellung von Kohlenmonoxid für die Synthese von Phosgen.
24. Verwendung von Material (2), enthaltend mindestens eine polymere, organische Verbindung, in einer partiellen Oxidation des Materials zur Bereitgestellung von Kohlenmonoxid für die Synthese von Phosgen.
25. Verwendung nach einem der Aspekte 23 oder 24, dadurch gekennzeichnet, dass die partielle Oxidation des Materials gemäß eines Verfahrens nach einem der Aspekte 1 bis 16 erfolgt.

### Beispiele

### Beispiel 1 - Erfindungsgemäßes Verfahren - partielle Oxidation 2 von Polyurethan-haltigem Material (zwei Reaktionszonen 2a und 2b) - ohne CO₂ Rückführung gemäß Fig.1

In eine Vorrichtung für eine partielle Oxidation **2** werden in einen Reaktor **2a** 10,94 t/h polyurethanhaltiges Material **1a** mit einer Zusammensetzung von 54,2 Gew.% Kohlenstoff, 8,78 Gew.% Wasserstoff, 35,7 Gew.% Sauerstoff und 1,1 Gew.% N₂ sowie 0,5 Gew.% nicht verbrennbare anorganische Feststoffe eingebracht. Die Umsetzung des polyurethanhaltigen Materials **1a** erfolgt im Reaktor **2a** bei 950°C, woraus ein CO- und H₂-haltiges Produktgas **2c** resultiert, das in einen Reaktor **2b** geführt wird. In Reaktor **2b** erfolgt die Umsetzung des aus **2a** eingetragenen Gasgemisches **2c** bei 1450°C.

Weiterhin werden der partiellen Oxidation **2** 5,46 t/h Sauerstoff **22b** aus einer Wasserelektrolyse **22** zugeführt.

Das CO- und H₂ haltige Gasgemisch **2d** aus der Zone 2 **2b** wird in einem Waschschritt **3** mit Wasser kontaktiert und auf 60°C abgekühlt. Dabei werden 0,049 t/h Schlacke **3b** und 0,1 t/h Wasser sowie 0,54 t/h Asche abgetrennt. Eine Teilmenge des kondensierten Wassers wird gepurged und durch Frischwasser ersetzt.

Das gewaschene Produktgasgemisch **3a** aus der partiellen Oxidation **2** wird einer Trocknung **4** zugeführt.

Das getrocknete Produktgasgemisch **4a** wird einer Aminwäsche zur CO₂ Abtrennung **5** zugeführt, in der 7,9 t/h CO₂ **5b** abgetrennt werden. Das von CO₂ weitgehend befreite Produktgasgemisch **5a** wird einer Cold-box zur H₂-CO Trennung **6** zugeführt, bei der das restliche verbliebene CO₂ ebenfalls abgetrennt wird. Aus der Cold-box werden 6,28 t/h CO **6b** und 0,95 t/h H₂-haltiges Restgas **6a** und geringe Mengen Nebenprodukte unter anderem Methan und Stickstoff entnommen.

Das CO **6b** wird einer Phosgensynthese **8** zugeführt und dort mit 15,92 t/h Cl₂ zu 22,2 t/h Phosgen **8a** umgesetzt. Das Phosgen **8a** wird mit 13,68 t/h Toluoldiamin einer IsocyanatSynthese **9** zu 19,5 t/h Toluoldiisocyanat **9a** umgesetzt. Dieses kann mit Poly-/Polyesterolen **10a** zu Polyurethan-Werkstoffen weiter verarbeitet werden.

Nach der Nutzung der Polyurethan-Werkstoffe in verschiedenen Anwendung werden diese Endof-life Materialien **110** gesammelt, getrennt und aufbereitet, so dass ein Material **1b** entsteht, welches der Gasifikation **2** zugeführt werden kann. Somit kann die CO-Gruppe der funktionelle Isocyanat-gruppe (-NCO) im Isocyanat nachhaltig hergestellt und der Kohlenstoff recycelt werden, womit für diesen Kohlenstoff die Wertschöpfungskette geschlossen wird.

Der aus der Cold-box **6** abgetrennte Wasserstoff-haltige Restgasstrom **6a** wird einer Pressure-Swing Adsorption Einheit **7** zur Reinigung des Wasserstoffes zugeführt und daraus 0,9 t/h H₂ abgetrennt und der der Hydrierung von Dinitrotoluol **20** zugeführt wird. Hierbei werden 20,4 t/h Dinitrotoluol **21** mit den 0,9 t/h H₂ **7a** aus der Restgasbehandlung **7** sowie weiteren 0,445 t/h H₂ aus einer Wasserelektrolyse **22** zu 13,68 t/h Toluol-diamin **20a** umgesetzt..

Mit dem Koppelprodukt Wasserstoff, welches für die Hydrierung der Nitroverbindungen eingesetzt wird, kann ein Großteil des benötigen Wasserstoffes bereitgestellt werden, welcher dann nicht mehr via Wasserelektrolyse hergestellt werden muss und liefert damit ebenfalls einen nachhaltigen Beitrag.

Der aus der Wasserelektrolyse **22** als Koppelprodukt entnommene Sauerstoff **22b** mit 3,6 t/h wird dabei mit weiterem Sauerstoff von 1,86t/h der Gasifikation **2** zugeführt.

### Beispiel 2 Fig.1 - Erfindungsgemäßes Verfahren -partielle Oxidation von Polyurethan-haltigem Material (zwei Reaktionszonen 2a und 2b) mit CO₂ Rückführung

In eine Vorrichtung für eine partielle Oxidation **2** werden 10,94 t/h polyurethanhaltiges Material **1a** mit einer durchschnittlichen Zusammensetzung von 54,2 Gew.% Kohlenstoff, 8,73 Gew.% Wasserstoff, 35,5 Gew.% Sauerstoff, 1,1 Gew.% N₂ sowie 0,45 Gew.% nicht verbrennbare anorganische Feststoffe eingebracht. Die Umsetzung des polyurethanhaltigen Materials **1a** erfolgt in der Vorrichtung in einer Zone **2a** unter Zusatz von sauerstoffhaltigem Gas **22b** bei 950°C, aus ein CO- und H₂-haltiges Gasgemisch in eine Zone 2 **2b** geführt werden. In der Zone 2 **2b** erfolgt die Umsetzung des aus **2a** eingetragenen Produktgasgemisches **2c** bei 1450°C. Weiterhin werden der Gasifizierungsvorrichtung (2) 5,46 t/h Sauerstoff **22b** aus einer Wasserelektrolyse **22** sowie 7,91 t/h CO₂ **5b** aus der CO₂-Abtrenneinheit **5** zugeführt. Das CO- und H₂ haltige Gasgemisch **2d** wird in einem Waschschritt **3** durch Waschen mit Wasser auf 60°C abgekühlt. Dabei werden 0,049 t/h Schlacke **3b**, 0,1 t/h Wasser und 0,54 t/h Asche abgetrennt. Eine Teilmenge des kondensierten Wassers 3,23 t/h wird gepurged und 0,1 t/h Frischwasser zugesetzt.

Das Produktgasgemisch **3a** aus dem Waschschritt **3** wird nach dem Abkühlen einer Trocknung **4** zugeführt.

Das getrocknete Gasgemisch **4a** wird einer Aminwäsche zur CO₂ Abtrennung **5** zugeführt, in der 7,91 t/h CO₂ **5b** abgetrennt werden. Das von CO₂ weitgehend befreite Gasgemisch wird einer Cold-box zur H2-CO Trennung **6** zugeführt, bei der das restliche verbliebene CO₂ ebenfalls abgetrennt wird. Aus der Cold-box werden 11,31 t/h CO **6b** und 0,6 t/h H2-haltiges Restgas **6a** und geringe Mengen Nebenprodukte unter anderem Methan und Stickstoff entnommen. Das CO **6b** wird einer Phosgensynthese **8** zugeführt und dort mit 28,67 t/h Cl₂ zu 39,98 t/h Phosgen **8a** umgesetzt. Das Phosgen **8a** wird mit 24,63 t/h Toluoldiamin **20a** einer Isocyanat-Synthese **9** zu 35,13 t/h Toluoldiisocyanat **9a** umgesetzt. Dieses kann mit Poly-/Polyesterolen **10a** zu Polyurethan-Werkstoffen weiter verarbeitet werden. Nach der Nutzung der Polyurethan-Werkstoffe in verschiedenen Anwendung werden diese als end-of-life Material **11a** gesammelt, getrennt und aufbereitet **1,** so dass ein Material **1a** entsteht, welches der partiellen Oxidation **2** zugeführt werden kann. Somit kann wird die CO-Gruppe der funktionelle Isocyanat-gruppe (-NCO) im Isocyanat nachhaltig hergestellt und der Kohlenstoff recycelt werden, womit für diesen Kohlenstoff die Wertschöpfungskette geschlossen wird.

Der aus der Cold-box 6 abgetrennte Wasserstoff-haltige Restgasstrom **6a** wird einer Pressure-Swing Adsorption Einheit 7 zur Reinigung des Restgases zugeführt und daraus 0,55t/h H₂ **7a** abgetrennt der der Hydrierung von Dinitrotoluol **20** zugeführt wird. Hierbei werden 34,74 t/h Dinitrotoluol **21** mit den 0,55t/h H₂ **7a** aus der Wasserstoffreinigungseinheit sowie weiteren 1,87 t/h H₂ aus einer Wasserelektrolyse **22** zu 24,63 t/h Toluol-diamin **20a** umgesetzt.

Mit dem Koppelprodukt Wasserstoff, welches für die Hydrierung der Nitroverbindungen eingesetzt wird, kann ein Großteil des benötigen Wasserstoffes bereitgestellt werden, welcher dann nicht mehr via Wasserelektrolyse hergestellt werden muss und stellt damit ebenfalls ein nachhaltigen Beitrag.

Von den bei der Wasserelektrolyse **22** als Koppelprodukt entstandenen Menge Sauerstoff **22b** von 14,97 t/h werden 5,46 t/h der partiellen Oxidation **2** zugeführt

### Beispiel 3, Fig.2 - Erfindungsgemäßes Verfahren - Gasifizierung im Polycarbonatverbund - ohne CO₂ Rückführung

In einer Gasifizierungsvorrichtung **2** werden 10,935 t/h polycarbonat-haltiges Material **1b** mit einer durchschnittlichen Zusammensetzung von 75,65 Gew.% Kohlenstoff, 5,51 Gew.% Wasserstoff, 18,9 Gew.% Sauerstoff, sowie 0,45 Gew.% nicht verbrennbare anorganische Feststoffe eingebracht. Die Umsetzung des polycarbonat-haltigen Materials **1b** erfolgt in der Gasifizierungsvorrichtung 2 in einer Zone **2a** bei 950°C, aus ein CO- und H2-haltiges Produktgasgemisch **2c** in eine Zone 2 **2b** geführt werden sowie 0,54 t/h Asche ausgetragen werden (nicht gezeichnet). In **2b** erfolgt die Umsetzung des aus **2a** eingetragenen Gasgemisches **2c** bei 1450°C. Weiterhin werden der Gasifizierungsvorrichtung 9,89 t/h Sauerstoff **22b** aus einer Wasserelektrolyse **22** zugeführt. Das CO- und H₂ haltige Produktgasgemisch **2d** aus **2b** wird in einem Waschschritt **3** durch Waschen mit Wasser auf 60°C abgekühlt. Dabei werden 0,049 t/h Schlacke **3b** und 0,1 t/h Wasser abgetrennt. Eine Teilmenge von 1,6 t/h des kondensierten Wassers wird gepurged und durch 1,7 t/h Frischwasser ersetzt.

Das abgekühlte Produktgasgemisch **3a** aus Waschschritt **3** wird einer Trocknung **4** zugeführt.

Das getrocknete Produktsgasgemisch **4a** wird einer Aminwäsche zur CO₂ Abtrennung **5** zugeführt, in der 6,52 t/h CO₂ **5b** abgetrennt werden. Das von CO₂ weitgehend befreite Gasgemisch **5a** wird einer Cold-box zur H2-CO Trennung **6** zugeführt, bei der das restliche verbliebene CO₂ ebenfalls abgetrennt wird. Aus der Cold-box werden 12,61 t/h CO **6b** und 0,6 t/h H₂-haltiges Restgas **6a** und geringe Mengen Nebenprodukte unter anderem Methan entnommen.

Der aus der Cold-box abgetrennte Wasserstoff-haltige Restgasstrom **6a** wird einer Pressure-Swing Adsorption Einheit 7 zur Reinigung des Wasserstoffes zugeführt und daraus 0,55 t/h H₂ **7a** abgetrennt und einer weiteren Verwertung zugeführt.

Das CO aus der Cold-box wird einer Phosgensynthese **8** zugeführt und dort mit 31,97 t/h Cl₂ zu 44,58 t/h Phosgen **8a** umgesetzt.

Aus der Phosgensysnthese **8** werden 44,58 t/h Phosgen **8a** entnommen und in einer Polycarbonat-Herstellung **100** als Lösung in chloriertem Lösungsmittel (z.B. 482 t/h eines 1:1 Gemisches aus Methylenchlorid und Chlorbenzol) (2) mit 599,4 t/h 15 Gewichtsprozentiger Bisphenol-A Lösung **102** (89,9 t/h BPA entsprechend 393,8 kmol) in alkalischem Wasser (mit 2,13 Mol NaOH pro Mol Bisphenol A, entsprechend 838,8 kmol NaOH = 33,5 t/h NaOH und 71,2 t H2O als 32 %ige Natronlauge)) zu 102,9 t/h Polycarbonat **101** umgesetzt mittels Einsatz mindestens eines Kettenabbrechers (z.B. 2,55 t/h tert-Butylphenol), 32,19 t/h weiterer 22 Gewichtsprozentiger NaOH-Lösung (7,08 t/h NaOH und 19,8 t/h H2O als 32%ige Natronlauge) und mindestens ein Katalysator (z.B. 0,5 t/h N-Ethylpiperidin). Dabei werden 36,0 t/h NaOH mit 76,5 t/h Wasser als 32 Gew.%ige Natronlauge **200a** aus der Chlor-Alkalielektrolyse **200** bezogen. Eine zusätzliche Menge von 4,58 t/h NaOH mit 19,8 t/h H₂O wird extra zugeführt.

Bei der Polycarbonatproduktion werden 627,2 t/h Wasch- und Prozesswasser eingesetzt, das unter anderem das NaCl-haltigen Abwasser **200b** bildet.

Das dabei anfallende NaCl-haltige Abwasser wird mit einer Menge von 59,38 t/h NaCl und 1128,3 t/h Wasser als NaCl-haltiges Abwasser erhalten. Ein Teilstrom bestehend aus 0,85 t/h NaCl und 16,2 t/h H₂O **200b** wird nach Reinigung über eine Strippanlage mit Schleppgas und ein Aktivkohle Reinigung einer Cl₂-Herstellung durch Chloralkali-elektrolyse **200** zugeführt. Damit wird der Wasserbedarf der Elektrolyse gedeckt, womit der Zusatz von Wasser zur Chloralkali-Elektrolyse entfällt. Dies schont die Ressource Wasser. Das erhaltene Polycarbonat **101** wird als zur Darstellung von Polycarbonat-Material für die Anwendungen im Markt eingesetzt.

Nach Nutzung des Polycarbonat-Materials in diversen Anwendungen im Markt, kann dieses als end-of-life Material **110a** gesammelt und recycelt werden, um den daraus erhaltenen

Polycarbonat-Material Abfall nach Aufarbeitung **1** als Material zur partiellen Oxidation **1b** einer Gasifizierungsvorrichtung zuzuführen.

Durch das erfindungsgemäße Verfahren können 6,25% des Kohlenstoffs, der im PC vorhanden ist, aus einer nichtfossilen Kohlenstoffquelle ersetzt werden. Durch Einsatz von regenerativer Energie bei der Chloralkalielektrolyse wird der CO2-Footprint des aus CO und Cl2 hergestellten Phosgens weiterhin erniedrigt und ermöglicht so ein nachhaltige hergestelltes PC.

### Beispiel 4 Fig. 2 - Erfindungsgemäßes Verfahren - Gasifizierung im Polycarbonatverbund - mit CO₂-Rückführung

In einer Gasifizierungsvorrichtung **2** werden 10,935 t/h polycarbonat-haltiges Material **1b** mit einer durchschnittlichen Zusammensetzung von 75,65 Gew.% Kohlenstoff, 5,51 Gew.% Wasserstoff, 18,9 Gew.% Sauerstoff, sowie 0,45 Gew.% nicht verbrennbare anorganische Feststoffe eingebracht. Die Umsetzung des polyurethanhaltigen Materials **1b** erfolgt in der Gasifizierungsvorrichtung **2** in einer Zone **2a** bei 950°C, aus ein CO- und H2-haltiges Gasgemisch **2c** in eine Zone 2 **2b** geführt werden sowie 0,54 t/h Asche ausgetragen werden. In **2b** erfolgt die Umsetzung des aus **2a** eingetragenen Gasgemisches **2c** bei 1450°C. Weiterhin werden der Gasifizierungsvorrichtung **2** 9,89 t/h Sauerstoff aus einer Wasserelektrolyse und 6,52 t/h CO₂ aus der CO₂-Abtrenneinheit zugeführt. Das CO- und H2 haltige Gasgemisch aus der Zone 2 wird durch Quenschen mit Wasser auf 60°C abgekühlt. Dabei werden 0,049 t/h Schlacke und 0,1 t/h Wasser abgetrennt. Eine Teilmenge 2,67 t/h des kondensierten Wassers wird gepurged und 0,1 t/h Frischwasser zugesetzt.

Das Gasgemisch aus der Gasifizierungsvorrichtung **2** wird nach dem Abkühlen einer Trocknung zugeführt.

Das getrocknete Gasgemisch wird einer Aminwäsche zur CO₂ Abtrennung zugeführt, in der 6,52 t/h CO₂ abgetrennt werden. Das CO₂ wird der Gasifizierungsvorrichtung wieder zugeführt. Das von CO₂ weitgehend befreite Gasgemisch wird einer Cold-box zur H₂-CO Trennung zugeführt, bei der das restliche verbliebene CO₂ ebenfalls abgetrennt wird. Aus der Cold-box werden 16,77 t/h CO und 0,31 t/h H₂ und geringe Mengen Nebenprodukte unter anderem Methan entnommen.

Der aus der Cold-box **6** abgetrennte Wasserstoff-haltige Gasstrom wird einer Pressure-Swing Adsorption Einheit **7** zur Reinigung des Wasserstoffes zugeführt und daraus 0,28 t/h H₂ abgetrennt und einer weiteren Verwertung zugeführt.

Das CO **6b** aus der Cold-box **6** wird einer Phosgensynthese **8** zugeführt und dort mit 42,52 t/h Cl₂ zu 59,29 t/h Phosgen **8a** umgesetzt.

Aus der Phosgensysnthese **8** werden 44,58 t/h Phosgen **8a** entnommen und in einer Polycarbonat-Herstellung **100** als Lösung in chloriertem Lösungsmittel (z.B. 641 t/h eines 1:1 Gemisches aus Methylenchlorid und Chlorbenzol) (2) mit 797,2 t/h 15 Gewichtsprozentiger Bisphenol-A Lösung **102** (119,56 t/h BPA entsprechend 523,74 kmol) in alkalischem Wasser (mit 2,13 Mol NaOH pro Mol Bisphenol A, entsprechend 1115,6 kmol NaOH = 44,55 t/h NaOH und 94,69 t/h H2O als 32 %ige Natronlauge)) als Diol zu 136,85 t/h Polycarbonat **101** umgesetzt mittels Einsatz mindestens eines Kettenabbrechers (z.B. 3,39 t/h tert-Butylphenol), 42,8 t/h weiterer 22 Gewichtsprozentiger NaOH-Lösung (9,4 t/h NaOH und 26,3 t/h H2O als 32%ige Natronlauge) und mindestens ein Katalysator (z.B. 0,66 t/h N-Ethylpiperidin). Dabei werden 47,88 t/h NaOH mit 101,75 t/h Wasser als 32 Gew.%ige Natronlauge aus der Chlor-Alkalielektrolyse **200** bezogen. Eine zusätzliche Menge von 6,09 t/h NaOH mit 26,33 t/h H2O wird extern zugekauft und zugeführt.

Bei der Polycarbonatproduktion werden 834,17 t/h Wasch- und Prozesswasser eingesetzt, das unter anderem das NaCl-haltigen Abwasser bildet.

Das dabei anfallende NaCl-haltige Abwasser **200b** mit einer Menge von 78,97 t/h NaCl und 1500,6 t/h Wasser als NaCl-haltiges Abwasser erhalten. Ein Teilstrom bestehend aus 1,13 t/h NaCl und 21,55 t/h H2O wird nach Reinigung über eine Strippanlage mit Schleppgas und ein Aktivkohle Reinigung einer Cl2-Herstellung durch Chloralkali-Elektrolyse **200** zugeführt. Damit wird der Wasserbedarf der Elektrolyse gedeckt, womit der Zusatz von Wasser zur Chloralkali-Elektrolyse **200** entfällt. Dies schont die Ressource Wasser. Das erhaltene Polycarbonat **101** wird als zur Darstellung von Polycarbonat-Material für Anwendungen im Markt eingesetzt.

Nach Nutzung des Polycarbonat-Materials in diversen Anwendungen im Markt **110**, kann dieses gesammelt und recycelt werden, um den daraus erhaltenen Polycarbonat-Material Abfall nach Aufarbeitung **1** als Material zur partiellen Oxidation **1b** einer Gasifizierungsvorrichtung zuzuführen.

Durch das erfindungsgemäße Verfahren können 6,25% des Kohlenstoffs, der im Polycarbonat vorhanden ist, aus einer nichtfossilen Kohlenstoffquelle ersetzt werden. Durch Einsatz von regenerativer Energie bei der Chloralkalielektrolyse wird der CO₂-Footprint des aus CO und Cl₂ hergestellten Phosgens weiterhin erniedrigt und ermöglicht so ein nachhaltig hergestelltes Polycarbonat.

## Patentansprüche

**1.** Verfahren zur Bereitstellung von Kohlenmonoxid für die Herstellung von Phosgen, bevorzugt für die Herstellung von Phosgen zur Synthese organischer Isocyanatverbindung, enthaltend mindestens folgende Schritte
b) Bereitstellung eines sauerstoffhaltigen Gasstroms (22b), enthaltend mindestens 50 Gew.-% Sauerstoffgas,
b) partielle Oxidation (2) von Material (1a), enthaltend mindestens eine polymere, organische Verbindung, wobei besagtes Material (1a) in einen Reaktor (2a) einer Vorrichtung eingebracht und dort zumindest durch Zufuhr des besagten sauerstoffhaltigen Gasstromes (22b) und von Wärme bei mindestens 400°C unter Bildung eines Produktgases (2c) behandelt und das resultierende Produktgas (2c), gegebenenfalls nach mindestens einem weiteren partiellen Oxidationsschritt (2b) des Produktgases (2c), als ein Kohlenmonoxid-haltiger Produktgas-Strom (2d) gemeinsam mit darin dispergiertem, partikelförmigem Feststoff aus der Vorrichtung ausgebracht wird;
c) der Kohlenmonoxid-haltige Produktgas-Strom (2d) einer Reinigung zugeführt wird, in der zumindest
cl) der Kohlenmonoxid-haltige Produktgas-Strom (2d) zur Abtrennung von Feststoff einem Waschschritt (3) zugeführt wird, worin
(i) Wasser mit dem Kohlenmonoxid-haltigen Produktgas-Strom (2d) in Kontakt gebracht wird, wodurch der im Kohlenmonoxid-haltigen Produktgas-Strom (2d) dispergierte, partikelförmige Feststoff eine Schlacke (3b) bildet und diese Schlacke (3b) entfernt und abgeführt wird,
(ii) der von partikelförmigem Feststoff gereinigte, Kohlenmonoxid-haltige Produktgas-Strom (3a) ausgebracht wird;
c2) ein mittels Waschschritt (3) von partikelförmigem Feststoff gereinigter Kohlenmonoxid-haltiger Produktgas-Strom (3a) in einem Trockenschritt zumindest einer Abtrennung von Wasser (4) zugeführt, Wasser (4b) abgetrennt und der resultierende Kohlenmonoxid-haltige Produktgas-Strom (4a) ausgebracht wird,
c3) ein mittels Abtrennung von Wasser (4) gereinigter Kohlenmonoxid-haltiger Produktgas-Strom (4a) zumindest einer Abtrennung von Kohlendioxid (5) zugeführt, Kohlendioxid abgetrennt und der resultierende Kohlenmonoxid-haltige Produktgas-Strom (5a) und Kohlendioxid (5b) ausgebracht wird;
c4) ein mittels Abtrennung von Kohlendioxid gereinigter Kohlenmonoxid-haltiger Produktgas-Strom (5a) zumindest einer Trenneinheit (6) zur Abtrennung von Kohlenmonoxid zugeführt, eine Abtrennung von Kohlenmonoxid durchgeführt und das resultierende Kohlenmonoxid (6b) und ein Wasserstoffgas-haltiges Restgas (6a) ausgebracht wird;
c5) optional ein mittels besagter Trenneinheit (6) abgetrenntes, Wasserstoffgas-haltiges Restgas (6a) einer Restgasbehandlung (7) zugeführt, Wasserstoffgas (7a) abgetrennt und Wasserstoffgas (7a) und Endgas (7b) ausgebracht wird;
d) gegebenenfalls Phosgensynthese (8) durch Umsetzung von zumindest Chlorgas und Kohlenmonoxid, wobei als Kohlenmonoxid mindestens das Kohlenmonoxid (6b) aus der Reinigung des Schrittes c) umgesetzt und Phosgen (8a) ausgebracht wird.

**2.** Verfahren nach Anspruch 1, **dadurch gekennzeichnet, dass** zur Bereitstellung des sauerstoffhaltigen Gasstroms (22b) eine Elektrolyse von Wasser (22) unter Erhalt von Sauerstoffgas (22a) und Wasserstoffgas durchgeführt wird und das Sauerstoffgas (22a) aus dieser Elektrolyse (22) zur Bereitstellung des sauerstoffhaltigen Gasstromes (22b) genutzt wird.

**3.** Verfahren nach einem der Ansprüche 1 oder 2, **dadurch gekennzeichnet, dass** dem Reaktor (2a) zusätzlich gasförmiges Wasser zugeführt wird, in einem Gewichtsverhältnis von gasförmigem Wasser zu Sauerstoffgas des sauerstoffhaltigen Gasstromes von mindestens 0,2.

**4.** Verfahren nach einem der vorangehenden Ansprüche, **dadurch gekennzeichnet, dass** die partielle Oxidation (2) bei einem absoluten Druck von mehr als 1 bar, bevorzugt bei einem absoluten Druck in einem Bereich von 2 bis 80 bar, besonders bevorzugt bei einem absoluten Druck in einem Bereich von 2 bis 50 bar, durchgeführt wird.

**3.** Verfahren nach einem der vorangehenden Ansprüche, **dadurch gekennzeichnet, dass** die Gesamtmenge aller in dem Material (1a) enthaltenden polymeren, organischen Verbindungen mindestens 50 Gew.-%, bevorzugt von mindestens 60 Gew.-%, besonders bevorzugt von mindestens 75 Gew.-%, beträgt.

**4.** Verfahren nach einem der vorangehenden Ansprüche, **dadurch gekennzeichnet, dass** die Gesamtmenge aller in dem Material (1a) enthaltenden polymeren, organischen Verbindungen einen Kohlenstoffanteil von mindestens 40,0 Gew.%, bevorzugt von mindestens 50 Gew.-%, aufweist.

**5.** Verfahren nach einem der vorangehenden Ansprüche, **dadurch gekennzeichnet, dass** das Gewichtsverhältnis des im sauerstoffhaltigen Gasstrom (22b) enthaltenen Sauerstoffgases zur polymeren, organischen Verbindung innerhalb eines Gewichtsverhältnisbereiches von 0,4 zu 1,0 bis 1,2 zu 1,0, bevorzugt von 0,6 zu 1,0 bis 0,9 zu 1,0 in den Reaktor (2a) eingebracht werden.

**6.** Verfahren nach einem der vorangehenden Ansprüche, **dadurch gekennzeichnet, dass** die polymere, organische Verbindung des Materials (1a) ausgewählt wird aus mindestens einer homopolymeren oder copolymeren Verbindung aus der Gruppe Cellulose, Polyester, Polyamid, Polyurethan (PUR), Polyharnstoff, Polyisocyanurat (PIR), Polycarbonat, bevorzugt aus mindestens einer homopolymeren oder copolymeren Verbindung der Gruppe Polyurethan (PUR), Polyisocyanurat (PIR).

**7.** Verfahren nach einem der vorangehenden Ansprüche, **dadurch gekennzeichnet, dass** die Struktur der polymeren, organischen Verbindung des Materials (1a) mindestens eine sich wiederholende Struktureinheit der Formel (I) enthält, 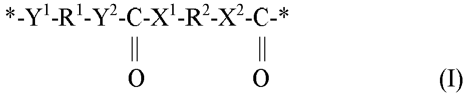 worin
X¹ und X² unabhängig voneinander eine direkte Bindung, O oder NH, bevorzugt O oder NH, bedeuten,
Y¹ und Y² unabhängig voneinander für O oder NH stehen,
R¹ und R² unabhängig voneinander jeweils für ein beliebiges bivalentes, Kohlenstoff enthaltendes Strukturelement stehen, und
eine mit ^{∗} gekennzeichnete kovalente Bindung eine Bindung zum Restmolekül der polymeren, organischen Verbindung bedeutet.

**8.** Verfahren nach Anspruch 7, **dadurch gekennzeichnet, dass** die Gesamtmenge der in dem Material (1a) enthaltenden polymeren, organischen Verbindungen mit mindestens einer sich wiederholenden Struktureinheit der Formel (I) mindestens 50 Gew.-%, bevorzugt von mindestens 60 Gew.-%, besonders bevorzugt von mindestens 75 Gew.-%, beträgt.

**9.** Verfahren nach einem der vorangehenden Ansprüche, **dadurch gekennzeichnet, dass** in die partielle Oxidation des Materials (1a) in dem Reaktor (2a) bei einer Temperatur in einem Temperaturbereich von 600 °C bis 1500°C, insbesondere von 850°C bis 1400°C, weiter bevorzugt von 1100°C bis 1300°C, durchgeführt wird.

**10.** Verfahren nach einem der vorangehenden Ansprüche, **dadurch gekennzeichnet, dass** der aus dem Waschschritt (3) ausgebrachte, von partikelförmigem Feststoff gereinigte, Kohlenmonoxid-haltige Produktgas-Strom (3a) eine Temperatur von höchstens 100 °C bevorzugt höchstens 80°C, hat.

**11.** Verfahren nach einem der vorangehenden Ansprüche, **dadurch gekennzeichnet, dass** die partielle Oxidation (2) des Materials (1a) in mindestens zwei Schritten durchgeführt wird, wobei in einem ersten Schritt eine partielle Oxidation des Materials (1a) in einem ersten Reaktor (2a) unter Zuführung eines sauerstoffhaltigen Gasstromes (22b) bei einer Temperatur in einem Temperaturbereich von 400 °C bis 800°C durchgeführt wird, das Produktgas (2c) des ersten Reaktors in einen zweiten Reaktor (2b) überführt wird und dort in einem weiteren Schritt unter Zuführung eines sauerstoffhaltigen Gasstromes (22b) einer partiellen Oxidation bei einer Temperatur von mehr als 800 °C, bevorzugt bei einer Temperatur in einem Bereich von 1300°C bis 1500°C, unterworfen wird unter Erhalt des Kohlenmonoxid-haltigen Produktgas-Stroms (2d).

**12.** Verfahren nach einem der Ansprüche 1 bis 12, **dadurch gekennzeichnet, dass** das in Schritt c3) ausgebrachte, abgetrennte Kohlendioxid (5b) zu einem Reaktor der partiellen Oxidation (2) zurückgeführt und in diesen Reaktor eingebracht wird.

**13.** Verfahren nach Anspruch 12, **dadurch gekennzeichnet, dass** die partielle Oxidation (2) des Materials (1a) gemäß Anspruch 11 durchgeführt wird und dass das in Schritt c3) ausgebrachte, abgetrennte Kohlendioxid (5b) zumindest zum ersten Reaktor (2a) der partiellen Oxidation zurückgeführt und in diesen Reaktor eingebracht wird.

**14.** Verfahren nach einem der vorangehenden Ansprüche, **dadurch gekennzeichnet, dass** der partiellen Oxidation (2) das Kohlendioxid (5b) in einer Menge von höchstens 1 kg pro kg polymerer, organischer Verbindung, bevorzugt von höchstens 0,9 kg pro kg polymerer, organischer Verbindung, besonders bevorzugt von 0,8 kg pro kg polymerer, organischer Verbindung, zugefügt wird.

**15.** Verfahren nach einem der vorangehenden Ansprüche, **dadurch gekennzeichnet, dass** die Phosgensynthese (8) in Schritt d) und zusätzlich folgender Schritt ausgeführt wird:
e) Herstellung von organischem Isocyanat (9), worin das Phosgen (8a) aus der Phosgensynthese (8) mit mindestens einer organischen Aminoverbindung (20a) umgesetzt und zumindest organisches Isocyanat (9a) ausgebracht wird.

**16.** Vorrichtung zur Bereitstellung von Kohlenmonoxid für die Herstellung von Phosgen, bevorzugt für die Herstellung von Phosgen zur Synthese organischer Isocyanatverbindung, enthaltend mindestens eine Dosiervorrichtung zur Einspeisung von Material, mindestens eine Vorrichtung zur partiellen Oxidation und mindestens eine Aufreinigungsvorrichtung für Kohlenmonoxid-haltiges Produktgas, **dadurch gekennzeichnet, dass**
i) besagte Vorrichtung zur partiellen Oxidation
mindestens einen Auslass für einen Kohlenmonoxid-haltigen Produktgas-Strom
und
mindestens einen Reaktor für die partielle Oxidation des Materials mit mindestens einem Heizelement enthält, das zur Temperierung des Reaktors auf eine Temperatur von mindestens 400°C geeignet ist, wobei der Reaktor mindestens einen Einlass für einen sauerstoffhaltigen Gasstrom und mindestens einen Einlass für Material und mindestens einen davon verschiedenen Auslass für Produktgas enthält, wobei der Einlass für einen sauerstoffhaltigen Gasstrom in Fluidverbindung mit einer Quelle eines sauerstoffhaltigen Gasstroms mit mindestens 50 Gew.-% Sauerstoffgas steht und der Auslass für Produktgas des Reaktors, gegebenenfalls zumindest über mindestens einen zwischengeschalteten Reaktor zur partiellen Oxidation des Produktgases, mit dem Auslass für den Kohlenmonoxid-haltigen Produktgas-Strom in Fluidverbindung steht; und,
ii) die Dosiervorrichtung und besagte Vorrichtung zur partiellen Oxidation derart zueinander angeordnet und ausgestaltet sind, dass die Dosiervorrichtung über mindestens eine Zuleitung mit dem Einlass für Material des Reaktors für die partielle Oxidation des Materials in Fluidverbindung steht; und
iii) eine Aufreinigungsvorrichtung für Kohlenmonoxid-haltiges Produktgas, welche mindestens einen Auslass für Kohlenmonoxid und mindestens einen Einlass für den Kohlenmonoxid-haltigen Produktgas-Strom enthält, wobei dieser Einlass in Fluidverbindung mit dem Auslass für den Kohlenmonoxid-haltigen Produktgas-Strom der Vorrichtung zur partiellen Oxidation steht, wobei die Aufreinigungsvorrichtung derart ausgestaltet ist, dass der Kohlenmonoxid-haltige Produktgasstrom zur Aufreinigung durch nachfolgend aufgeführte Module in der angegebenen Rangfolge hindurchgeführt werden kann
iii-1) mindestens eine Gaswasch-Vorrichtung, enthaltend mindestens einen Einlass für den Kohlenmonoxid-haltigen Produktgas-Strom, mindestens einen Einlass für Wasser, mindestens einen Waschbehälter, in dem Wasser und Kohlenmonoxid-haltigen Produktgas-Strom zusammengeführt werden, sowie mindestens einen Auslass für Kohlenmonoxid-haltigen Produktgas-Strom und mindestens einen Auslass für Schlacke, wobei der Waschbehälter und der Auslass für Schlacke in Fluidverbindung stehen und der Waschbehälter und der Auslass für Kohlenmonoxid-haltigen Produktgas-Strom in Fluidverbindung stehen;
iii-2) mindestens eine Trocken-Vorrichtung, enthaltend mindestens einen Einlass für Kohlenmonoxid-haltigen Produktgas-Strom, mindestens eine Kühlvorrichtung, die zur Abkühlung des Kohlenmonoxid-haltigen Produktgas-Stroms geeignet ist mindestens einen Auslass für Kohlenmonoxid-haltigen Produktgas-Strom sowie mindestens einen Auslass für flüssiges Kondensat,
iii-3) mindestens eine Vorrichtung zur Abtrennung von Kohlendioxid, enthaltend mindestens einen Einlass für Kohlenmonoxid-haltigen Produktgas-Strom, mindestens einen Auslass für Kohlenmonoxid-haltigen Produktgas-Strom sowie zusätzlich mindestens einen Auslass für Kohlendioxid,
iii-4) mindestens eine Trenneinheit zur Abtrennung von Kohlenmonoxid, enthaltend mindestens einen Einlass für Kohlenmonoxid-haltigen Produktgas-Strom, mindestens einen Auslass für Kohlenmonoxid sowie zusätzlich mindestens einen Auslass für Wasserstoffgas-haltiges Restgas.

**17.** Vorrichtung Anspruch 16, **dadurch gekennzeichnet, dass** mindestens ein Reaktor der Vorrichtung zur partiellen Oxidation einen Einlass für Kohlendioxid aufweist, der in einer Fluidverbindung mit dem Auslass für Kohlendioxid der Vorrichtung zur Abtrennung von Kohlendioxid steht, wodurch eine Rückführung des Kohlendioxids in diesen Reaktor der Vorrichtung zur partiellen Oxidation entgegen der Fließrichtung des Kohlenmonoxid-haltigen Produktgasstroms ermöglicht wird.

**18.** Vorrichtung nach einem der Ansprüche 16 oder 17, **dadurch gekennzeichnet, dass** besagte Vorrichtung zur partiellen Oxidation
mindestens einen Auslass für einen Kohlenmonoxid-haltigen Produktgas-Strom
und
einen ersten Reaktor für die partielle Oxidation des Materials mit mindestens einer Regelung enthält, die zur Temperatureinstellung des Reaktors auf eine Temperatur von mindestens 400°C geeignet ist, wobei der erste Reaktor mindestens einen Einlass für einen sauerstoffhaltigen Gasstrom und mindestens einen Einlass für Material und mindestens einen davon verschiedenen Auslass für Produktgas enthält, wobei der Einlass für einen sauerstoffhaltigen Gasstrom in Fluidverbindung mit einer Quelle eines sauerstoffhaltigen Gasstroms mit mindestens 50 Gew.-% Sauerstoffgas steht;
und
einen zweiten Reaktor für die partielle Oxidation des Produktgases mit mindestens einem Heizelement, das zur Temperierung des Reaktors auf eine Temperatur von mehr als 800°C geeignet ist, enthält, wobei der zweite Reaktor mindestens einen Einlass für einen sauerstoffhaltigen Gasstrom und mindestens einen Einlass für gegebenenfalls zwischenbehandeltes Produktgas des ersten Reaktors und mindestens einen davon verschiedenen Auslass für Produktgas enthält, wobei der der Auslass für Produktgas des ersten Reaktors mit dem Einlass des zweiten Reaktors für gegebenenfalls zwischenbehandeltes Produktgas des ersten Reaktors in Fluidverbindung steht, und der Einlass für einen sauerstoffhaltigen Gasstrom in Fluidverbindung mit einer Quelle eines sauerstoffhaltigen Gasstroms mit mindestens 50 Gew.-% Sauerstoffgas steht, und der Auslass für Produktgas des zweiten Reaktors mit dem Auslass für den Kohlenmonoxid-haltigen Produktgas-Strom in Fluidverbindung steht.

**19.** Vorrichtung nach einem der Ansprüche 16 bis 18, **dadurch gekennzeichnet, dass** der Auslass für Kohlenmonoxid der Trenneinheit für Kohlenmonoxid in Fluidverbindung mit dem Einlass eines Reaktors einer Vorrichtung zur Phosgensynthese steht.

**20.** Verwendung von Material (2), enthaltend mindestens eine polymere, organische Verbindung, in einer partiellen Oxidation des Materials zur Bereitgestellung von Kohlenmonoxid für die Synthese von Phosgen.
